# EUROPEAN PATENT APPLICATION

(11) **EP 4 141 094 A1**
(43) Date of publication of application: **01.03.2023**
(21) Application number: 20932298.1
(22) Date of filing: 20.04.2020
(51) Int. Cl.: C12M 1/00, B01L 3/00

(54) **LIQUID SPREADING DEVICE, LIQUID SPREADING METHOD, LIQUID SPREADING SYSTEM, COMBINATION DEVICE, AND LIQUID PASSAGE DEVICE**

(71) Applicant: MGI Tech Co., Ltd., Shenzhen, Guangdong 518083 (CN)
(72) Inventor: CUI, Xingye, Shenzhen, Guangdong 518083 (CN); YANG, Joonmo, Sanjose, CA California 95134 (US); XU, Hong, Shenzhen, Guangdong 518083 (CN); SUI, Xiangkun, Shenzhen, Guangdong 518083 (CN); BOGDAN, Greg, San Jose, CA California 95134 (US)
(74) Representative: Zaboliene, Reda
(86) International application number: PCT/CN2020/085635
(87) International publication number: WO 2021/212276

(57) **Abstract**

A fluid laying device includes a manifold block. The manifold block defines a first channel, and also defines a first fluid inlet, a first fluid outlet, and several carrier interfaces. Each carrier interface has a first hole connecting one of a channel inlet and channel outlets of the flow cell carrier. The manifold block is provided with an inlet valve device, a bypass valve device, and outlet valve devices. The inlet valve device and the outlet valve devices are one-to-one corresponding to the carrier interfaces, and disposed on a fluid path from the corresponding carrier interface to the first channel. The first channel includes a first section and a second section. A bypass valve device is between the first and second sections to control connection and disconnection between the first and second sections. The inlet valve device connects the first section, and the outlet valve devices connect the second section.

## Description

### FIELD

The subject matter relates to a fluid laying device, a fluid laying method, a fluid laying system, a composite device, and a fluid passage device.

### BACKGROUND

Fluid laying technology on a solid surface allows an even spread of fluid on a solid carrier. Such a technology is commonly used on non-enclosed carriers and includes spraying, atomization deposition, and coating. The uniformity of a fluid layer laid down by the above fluid laying technology mainly depends on the performance or working mode of an external spraying device, and the flow factor or viscosity of the internal fluid is basically not an important factor. However, for the fluid laying technology to work efficiently in closed carriers, especially in carriers which include a channel with a fluid inlet and a fluid outlet, an inside channel may not be reached by fluid under external effects. In such circumstances, the fluid flow factors become important during fluid laying.

With the development of microfluidic technology in recent years, the research on fluid laying technology in carriers having channels has gradually increased. Pressure-driven is widely used in fluid laying because of its high controllability and ease of use. At present, a carrier widely available may have a channel structure with a large aspect ratio (a ratio of long side to short side), which has a low requirement for uniformity of fluid laying. A carrier with large aspect ratio generally requires a large movement space in a certain direction for a signal acquisition device to acquire signals indicate a large directional movement, which is not conducive to centralization or miniaturization of the signal acquisition device. In contrast, in this respect, a carrier with a small aspect ratio (such as square), has some advantage. However, an effective fluid laying method, device, and system are still absent in relation to a carrier with large surface area and small aspect ratio.

In addition, high-throughput sequencing is required in the commercial sequencing field, the advantages of the carrier with large surface area in realizing high-throughput sequencing tasks are clear. Designing and developing fluid laying or fluid passing methods suitable for a carrier with large surface area, especially developing fluid laying methods with controllable uniformity, is critical to the improvement of the throughput, performance, and quality of sequencing and the reduction of sequencing costs. Therefore, the demand for effective fluid laying technology for the carrier with large surface area is particularly urgent.

### SUMMARY

In order to solve some or all of the above problems and other potential problems in the related art, a fluid laying device, a fluid laying method, a fluid laying system, a composite device, and a fluid passage device are needed.

In a first aspect, a fluid laying device for laying fluid on a flow cell carrier is provided. The fluid laying device includes a manifold block. The manifold block defines a first channel, the manifold block includes a first fluid inlet communicating with the first channel, a first fluid outlet, and a plurality of carrier interfaces. Each of the plurality of carrier interfaces defines a first hole, the first hole of each of the plurality of carrier interfaces is configured to connect one of a channel inlet and a channel outlet of the flow cell carrier. An inlet valve device, a bypass valve device, and a plurality of outlet valve devices are provided on the manifold block. The inlet valve device and the plurality of outlet valve devices correspond to the plurality of carrier interfaces one-to-one, and are disposed on fluid paths from the corresponding carrier interfaces to the first channel for opening or closing the fluid paths. The first channel includes a first section and a second section, the bypass valve device is between the first section and the second section to control connection or disconnection between the first section and the second section, the inlet valve device is connected to the first section, and the plurality of outlet valve devices is connected to the second section.

Furthermore, the fluid laying device further includes a support table and a carrier mounting table, the carrier mounting table and the manifold block are disposed on the support table, and the carrier mounting table is configured to mount the flow cell carrier.

Furthermore, the carrier mounting table is configured to adsorb the flow cell carrier by vacuum adsorption or low-pressure adsorption, and/or, the manifold block surrounds the carrier mounting table.

Furthermore, the plurality of carrier interfaces disconnecting from the channel inlet and the channel outlet of the flow cell carrier allows a fluid in the first channel to pass through the first hole of the corresponding carrier interface and enter the second hole of the corresponding carrier interface, and to finally flow out from the second fluid outlet through the second channel.

Furthermore, the manifold block further defines a second channel, the manifold block further includes a second fluid outlet communicating with the second channel, each of the plurality of carrier interfaces defines a second hole, and the second hole of each of the plurality of carrier interfaces is configured to communicate with the second channel.

Furthermore, the manifold block further defines a second fluid inlet communicating with the second channel, each of the plurality of carrier interfaces includes at least two second holes, the second channel is divided into a plurality of sections by the plurality of carrier interfaces, adjacent two of the plurality of sections communicate with each other through the second holes of the corresponding carrier interface, thereby allowing the fluid from the second fluid inlet to flow out from the second fluid outlet after passing through the plurality of carrier interfaces in sequence.

Furthermore, the manifold block further defines a second channel and a third channel, the manifold block further includes a second fluid inlet communicating with the second channel and a second fluid outlet communicating with the third channel, each of the plurality of carrier interfaces has at least two second holes, two of the at least two second holes communicate with the second channel and the third channel, thereby allowing a fluid from the second fluid inlet to flow out from the second fluid outlet after passing through the plurality of carrier interfaces in sequence.

Furthermore, a sealing ring is arranged in each of the plurality of carrier interfaces, and each sealing ring defines a first hole communicating the first hole of the corresponding carrier interface.

Furthermore, each sealing ring further defines a second hole communicating with the second hole of the corresponding carrier interface, the second hole of the sealing ring allows the fluid from the first hole of the sealing ring to enter the second hole of the corresponding carrier interface.

Furthermore, each sealing ring includes a central portion and a ring body sleeved on the central portion, the ring body abuts against a wall of the corresponding carrier interface, the first hole of the sealing ring is defined on the central portion, and the second hole is defined on the ring body and communicates an upper side and a lower side of the ring body with each other.

Furthermore, each sealing ring includes a central portion, a ring body, and a connecting portion connecting the ring body and the central portion, the ring body abuts against a wall of the corresponding carrier interface, the first hole of the sealing ring is defined on the central portion, and the second hole is defined on the connecting portion and communicates an upper side and a lower side of the connecting portion with each other.

Furthermore, the fluid laying device further includes the flow cell carrier, the flow cell carrier includes a base and a cover, a channel is formed between the base and the cover, and a portion of the manifold block constitutes the cover.

Furthermore, the portion of the manifold block constituting the cover defines the channel inlet and the channel outlet, and the channel inlet and the channel outlet communicate with the channel.

Furthermore, at least one groove for rectification of fluid is defined on a side of the manifold block facing the base, the at least one groove communicates with the channel inlet and at least one of the channel outlet, and/or the groove communicates with a portion or all of the channel outlet.

Furthermore, the portion of the manifold block constituting the cover defines one channel inlet and three channel outlets, any three of the fluid inlet and the fluid outlets are not on a same straight line.

Furthermore, the flow cell carrier is square, and the channel inlet and the channel outlets are distributed at four corners of the flow cell carrier.

Furthermore, the at least one groove for rectification of fluid includes two grooves, one of the two grooves communicates with the channel inlet and one of the channel outlets adjacent to the channel inlet, and the other of the two grooves communicates with another of the channel outlets adjacent to the channel inlet and a remaining channel outlet disposed diagonally opposite the channel inlet; or, the at least one groove for rectification of fluid is L-shaped and communicates with the three channel outlets.

Furthermore, the manifold block includes a plurality of sub-blocks, and each of the plurality of sub-block communicates with the corresponding channel through a pipeline.

Furthermore, the fluid laying device is configured to lay a nucleic acid sample or a reagent in the flow cell carrier.

In a second aspect, a fluid laying method is provided that configured to control the above fluid laying device to introduce a fluid into a channel in a flow cell carrier. The method including:
controlling a power device to start, thereby allowing the power device to push the fluid into the fluid inlet of the fluid laying device;
controlling the bypass valve device to open, thereby allowing the fluid to flow from the fluid inlet to the fluid outlet;
controlling the bypass valve device to close; and
controlling at least one of the inlet valve device and the plurality of outlet valve devices to open, thereby allowing the fluid to enter and flow through the channel in the flow cell carrier.

Furthermore, a different one of the plurality of outlet valve devices is controlled to open or the plurality of outlet valve devices is controlled to open in sequence, thereby controlling a direction of the fluid in the flow cell carrier, so that the fluid lays at all positions of the channel in sequence.

Furthermore, a corresponding one of the plurality of outlet valve devices is controlled to open or the plurality of outlet valve devices is controlled to open in a specific order, according to distribution and extension direction of grooves for rectification of fluid in the flow cell carrier.

Furthermore, includin two grooves for rectification of fluid in the flow cell carrier, a first groove communicates with the channel inlet and one of the channel outlets adjacent to the channel inlet, a second groove communicates with another of the channel outlets adjacent to the channel inlet and a remaining channel disposed diagonally opposite to the channel inlet, the specific order of controlling the plurality of outlet valve devices to open includes: first controlling the outlet valve device corresponding to the channel outlet communicating with the first groove to open; then controlling the outlet valve device corresponding to the channel outlet disposed diagonally opposite to the channel inlet to open; finally controlling the outlet valve device corresponding to the channel outlet communicating with the second groove to open.

Furthermore, when the L-shaped groove in the flow cell carrier communicates with the three channel outlets, one of the plurality of outlet valve devices controlled to be open is the outlet valve device corresponding to the channel outlet at an apex position of the L-shaped groove.

Furthermore, the fluid laying method is configured to spread a nucleic acid sample or a reagent in the flow cell carrier.

Furthermore, the fluid laying method uses a positive pressure to push the fluid to realize fluid laying, and uses a negative pressure to collect a waste fluid.

In a third aspect, a fluid laying system is provided, which includes:
the above fluid laying device;
a first power device configured to drive a fluid from a fluid source to enter and flow in the fluid laying device;
a valve device disposed between the power device and the fluid laying device, and configured to open or close a fluid path between the power device and the fluid laying device; and
a control device configured to control the power device, the valve device, and the inlet valve device and the plurality of outlet valve devices in the fluid laying device.

Furthermore, the fluid laying system further includes a second power device. The second power device is arranged downstream of the fluid laying device, and is configured to provide power for outflow of the waste fluid in the fluid laying device.

In a third aspect, a composite device is provided, which includes:
the above fluid laying device; and
an auxiliary cleaning tool, the auxiliary cleaning tool including a plurality of cleaning structures, each of the plurality of cleaning structures configured to align with and install on one carrier interface of the fluid laying device, and guide the fluid in the first hole of the carrier interface into the second hole of the carrier interface.

Furthermore, the auxiliary cleaning tool further includes a body, the plurality of cleaning structures is provided on the body, when the body is installed on the fluid laying device, the plurality of cleaning structures corresponds to the plurality of carrier interfaces.

Furthermore, the cleaning structure is cap-shaped, and includes a top portion and a flange portion extending from an edge of the top portion toward the fluid laying device, a guiding structure is provided on an inner side of the top portion for guiding the fluid flowing out of the first hole of the carrier interface into the second hole of the carrier interface; or, a guiding groove is defined on an inner side of the top portion for guiding the fluid flowing out of the first hole of the carrier interface into the second hole of the carrier interface; or, a guiding groove being I-shaped, cross-shaped, or resembling the Union Jack flag is defined on an inner side of the top portion for guiding the fluid flowing out of the first hole of the carrier interface into the second hole of the carrier interface.

Furthermore, the cleaning device is fixed to the body by adhesion or engagement.

A fluid passage device is also provided, which includes a manifold block. The manifold block defines a channel, the manifold block includes a fluid inlet communicating with the channel, a fluid outlet, and a plurality of carrier interfaces. The plurality of carrier interfaces configured to communicate a channel in the flow cell carrier when the flow cell carrier is installed on the fluid passage device. Each of the plurality of carrier interfaces has a first hole and a second hole, the second hole in each of the plurality of carrier interfaces is connected to the channel through a fluid path, the first hole in each of the plurality of carrier interfaces is connected to the channel through another fluid path, the first hole is configured to communicate with the channel in the flow cell carrier when the flow cell carrier is mounted on the fluid passage device.

The fluid entering the channel from the fluid inlet enters and exits the flow cell carrier through the first hole of each of the plurality of carrier interfaces, and then flows out through the fluid outlet; or, the fluid entering the channel from the fluid inlet enters the plurality of carrier interfaces through the first holes of the plurality of carrier interfaces, then enters the channel through the second holes of the plurality of carrier interfaces, and flows out from the fluid outlet; or, the fluid entering the channel from the fluid inlet enters and exits the plurality of carrier interfaces through the second holes, and then flows out through the fluid outlet.

Furthermore, a sealing ring is provided in each of the plurality of carrier interfaces, the sealing ring defines a first hole and a second hole, the first hole of the sealing ring communicates with the first hole of the corresponding carrier interface, and is used to communicate with the channel in the flow cell carrier, the second hole of the sealing ring allows the fluid from the first hole of the sealing ring to enter the second hole of the corresponding carrier interface.

Furthermore, the sealing ring includes a central portion and a ring body sleeved on the central portion, the ring body abuts against a wall of the corresponding carrier interface, the first hole of the sealing ring is defined on the central portion, the second hole is defined on the ring body and communicates an upper side and a lower side of the ring body with each other.

Furthermore, the sealing ring includes a central portion, a ring body, and a connecting portion connecting the ring body and the central portion, the ring body abuts a wall of the corresponding carrier interface, the first hole of the sealing ring is defined on the central portion, the second hole is defined on the connecting portion and communicates an upper side and a lower side of the connecting portion with each other.

Furthermore, the channel includes a first channel communicating with the fluid inlet and a second channel communicating with the fluid outlet, a first hole of the carrier interface communicates with the first channel, and a second hole of the carrier interface communicates with the second channel.

Furthermore, the fluid outlet includes a first fluid outlet and a second fluid outlet, the channel includes a first channel communicating with the fluid inlet and the first fluid outlet and a second channel communicating with the second fluid outlet, the first hole of the carrier interface communicates with the first channel, the second hole of the carrier interface communicates with the second channel.

Furthermore, the fluid inlet includes a first fluid inlet and a second fluid inlet, the fluid outlet includes a first fluid outlet and a second fluid outlet, the channel includes a first channel communicating the first fluid inlet and the first fluid outlet, and a second channel communicating the second fluid inlet and the second fluid outlet, the first hole of the carrier interface communicates with the first channel, the second hole of the carrier interface communicates with the second channel.

Furthermore, the fluid inlet includes a first fluid inlet and a second fluid inlet, the fluid outlet includes a first fluid outlet and a second fluid outlet, the channel includes a first channel communicating the first fluid inlet and the first fluid outlet, a second channel communicating the second fluid inlet, and a third channel communicating the second fluid outlet, the first hole of the carrier interface communicates with the first channel, each of the plurality of carrier interface includes a plurality of second holes, at least one of the plurality of second holes of the carrier interface communicates with the second channel, at least another of the plurality of second holes communicates with the third channel.

Furthermore, different sections of at least one of the channel communicate with each other via the second hole of the carrier interface.

Furthermore, one of the channel includes at least two sections, each of the plurality of carrier interface includes a plurality of second holes, at least one of the plurality of second holes of the carrier interface communicates with one of the sections, and at least another of the plurality of second holes of the carrier interface communicates with another of the sections.

Furthermore, the manifold block includes a plurality of sub-blocks, and each of the plurality of sub-blocks communicates with the corresponding channel through a pipeline.

The fluid laying device, fluid laying method, and fluid laying system provided in the present disclosure uses a positive pressure for laying of fluid to significantly improve the fluid laying speed and reduce the possibility of bubbles on the flow cell carrier. They can be applied to the fluid laying of flow cell carriers with large size and small aspect ratio, and can significantly improve the efficiency of replacement among reagents that enter the flow cell carriers in sequence and the uniformity of a fluid layer. The cleaning device and method provided increase the maintainability and reliability of the fluid laying device, significantly improve the service life of the device, and reduce the failure rate of the device.

### BRIEF DESCRIPTION OF THE DRAWINGS

Implementations of the present technology will now be described, by way of embodiment, with reference to the attached figures. Obviously, the drawings are only some embodiments of the present disclosure. For those of ordinary skill in the art, other drawings can be obtained based on these drawings without creative work.
FIG. 1A is a diagrammatic view of a flow cell carrier according to a first embodiment of the present disclosure.
FIG. 1B is a diagrammatic view of a flow cell carrier according to a second embodiment of the present disclosure.
FIG. 2 is a diagrammatic view of a fluid laying device according to a first embodiment of the present disclosure.
FIG. 3 is an exploded view of the fluid laying device of FIG. 2.
FIG. 4 is a diagrammatic view showing distributions of channels in a manifold block of the fluid laying device of FIG. 2.
FIG. 5 is a diagrammatic view showing flow directions of fluid in the manifold block of FIG. 4.
FIG. 6 is a diagrammatic view showing flow directions of fluid in the manifold block and the flow cell carrier of FIG. 4.
FIGS. 7A to 7C are diagrammatic views showing fluid laying processes in a flow cell carrier according to an embodiment of the present disclosure.
FIG. 8 is a perspective view of an auxiliary cleaning tool according to an embodiment of the present disclosure.
FIG. 9 is a perspective view of a cleaning device according to an embodiment of the present disclosure.
FIG. 10A is a perspective view of a sealing ring according to an embodiment of the present disclosure.
FIG. 10B is a partial cross-sectional view of the sealing ring of FIG. 10A.
FIG. 11 is a diagrammatic view showing distributions of channels in a manifold block of a fluid laying device according to a second embodiment of the present disclosure.
FIG. 12 is a diagrammatic view showing distributions of channels in a manifold block of a fluid laying device according to a third embodiment of the present disclosure.
FIG. 13 is a diagrammatic view of a fluid laying system according to a first embodiment of the present disclosure.
FIG. 14 is a diagrammatic view of a fluid laying system according to a second embodiment of the present disclosure.
Specific embodiments will further explain the present disclosure in combination with the above drawings.

Symbols of main components:
Flow cell carrier 1a, 1b, 72, or 82; channel 11a or 11b;
base 13a or 13b; cover 15a or 15b;
channel inlet 111a or 111b; channel outlet 113a or 113b;
groove 115a or 115b; fluid laying device 2, 5, 6, or 71;
manifold block 21, 41, 51, or 61; support table 22 or 42;
carrier mounting table 23 or 43; gas channel 231;
vacuum adapter 232; temperature control module 24;
temperature detector 25; first channel 211, 511, or 611;
second channel 212, 512, or 612a; fluid inlet 213 or 414;
first fluid outlets 214, 514, or 614; second fluid outlet 216, 516, or 616;
valve connection port 217, 517, or 617; valve device 218, 618, 751, 752, or 85;
inlet valve device 218a; outlet valve device 218b;
bypass valve device 218c; carrier interface 219, 413, 518, or 619;
sealing ring 26, 44, 52, or 62; first hole 261, 2191, 441, 4131, 521, or 621;
second hole 262, 2192, 442, 4132, or 522; central portion 263 or 443;
ring body 264 or 444; auxiliary cleaning tool 31;
cleaning structure 311; body 310;
positioning structure 312; mounting structure 313;
top portion 3111; flange portion 3113;
guiding structure 3115; cleaning device 4;
first section 411; second section 412;
connecting portion 445; fluid outlet 415;
joint 416; first fluid inlet 513 or 613;
second fluid inlet 515 or 615; third channel 612b;
fluid laying system 7 or 8; fluid source 73 or 83;
reagent fluid source 731; cleaning fluid source 733;
power device 741, 742, 743, 841, or 842; waste fluid storage unit 76 or 86;
pneumatic unit 77 or 87; control device 78 or 88;
functional device 81.

### DETAILED DESCRIPTION

Implementations of the disclosure will now be described, by way of embodiments only, with reference to the drawings. The described embodiments are only portions of the embodiments of the present disclosure, rather than all the embodiments. The disclosure is illustrative only, and changes may be made in the detail within the principles of the present disclosure. It will, therefore, be appreciated that the embodiments may be modified within the scope of the claims.

It should be noted that when a component is considered to be "disposed on" another component, the component can be arranged directly on another component, or there may be intermediate components therebetween. The term "and/or" as used herein includes all or any combination of one or more related listed items.

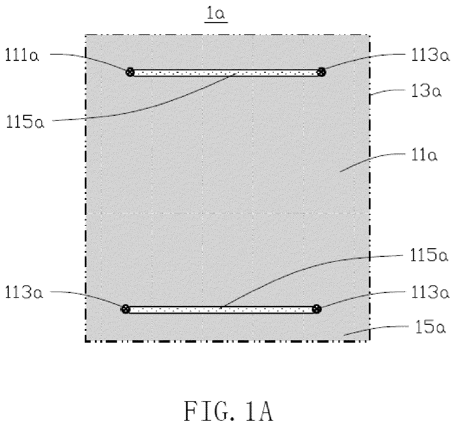

Referring to FIG. 1A, a flow cell carrier with a small aspect ratio is shown according to a first embodiment of the present disclosure. In the embodiment, the flow cell carrier 1a is square in shape, and has a square channel 11a inside. The flow cell carrier 1a has a base 13a on the lower side and a cover 15a on the upper side. The cover 15a covers the base 13a, and the channel 11a is formed between the cover 15a and the base 13a. Openings are defined on four corners of the base 13a. One of the openings is a channel inlet 111a, the other three of the openings are channel outlets 113a. Sealing rings (not shown) are provided outside the channel inlet 111a and the channel outlets 113a. A groove 115a is defined between the channel inlet 111a and one channel outlet 113a adjacent thereto (hereinafter referred to as a first channel outlet 113a). Another groove 115a is defined between the other two channel outlets 113a. The two grooves 115a are defined on a side of the base 13a facing the channel 11a. The two grooves 115a are used for rectification of fluids. One of the channel outlets 113a is disposed diagonally opposite the channel inlet 111a (hereinafter referred to as a second channel outlet 113a). The other channel outlet 113a (hereinafter referred to as a third channel outlet 113a) is adjacent to the channel inlet 111a. The first channel outlet 113a and the third channel outlet 113a are disposed at two sides of a line connecting the channel inlet 111a to the second channel outlet 113a.

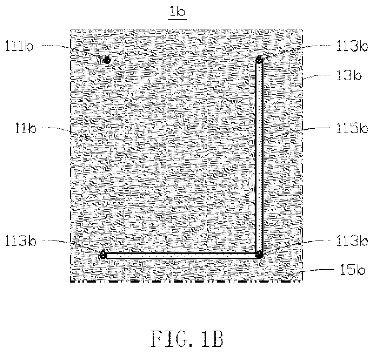

Referring to FIG. 1B, a flow cell carrier with a small aspect ratio is shown according to a second embodiment of the present disclosure. In the embodiment, the flow cell carrier 1b is also square, and has a square channel 11b inside. The flow cell carrier 1b has a base 13b on the lower side and a cover 15b on the upper side. The cover 15b covers the base 13b, and the channel 11b is formed between the cover 15b and the base 13b. Openings are defined at four corners of the base 13b. One of the openings is a channel inlet 1 11b, and the other three of the openings are channel outlets 113b. Sealing rings (not shown) are provided outside the channel inlet 111b and the channel outlet 113b. An L-shaped groove 115b for fluid rectification is defined by the three channel outlets 113b. The L-shaped groove 115b extends from one of the channel outlets 113b adjacent to the channel inlet 111b (hereinafter referred to as a first channel outlet 113b) to the channel outlet 113b disposed diagonally opposite the channel inlet 111b (hereinafter referred to as a second channel outlet 113b), and then extends from the second channel outlet 113b to the other channel outlet 113b adjacent to the channel inlet 111b (hereinafter referred to as a third channel outlet 113b). The second channel outlet 113b is disposed at an apex of the L-shaped groove 115b.

The L-shaped groove 115b is formed on a surface of the base 13b facing the channel 11b. The first channel outlet 113b and the third channel outlet 113b are disposed at two sides of a connecting line connecting the channel inlet 111b to the second channel outlet 113b.

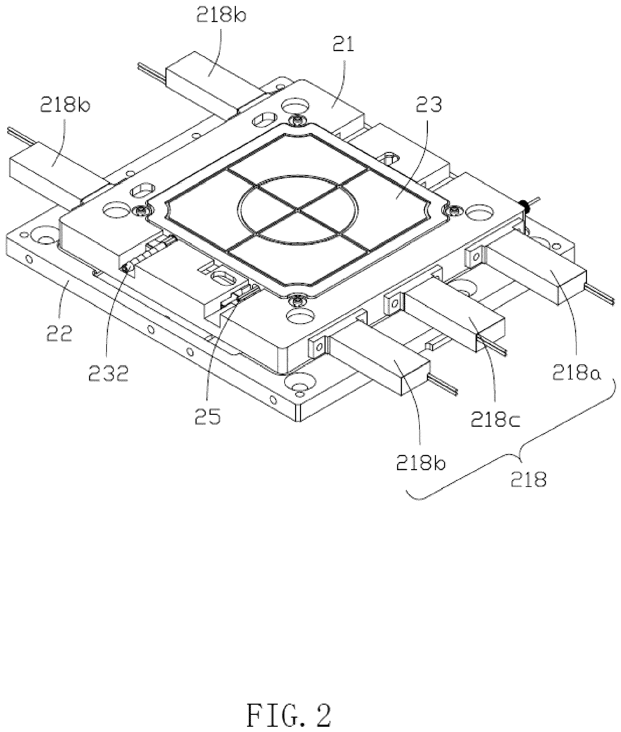

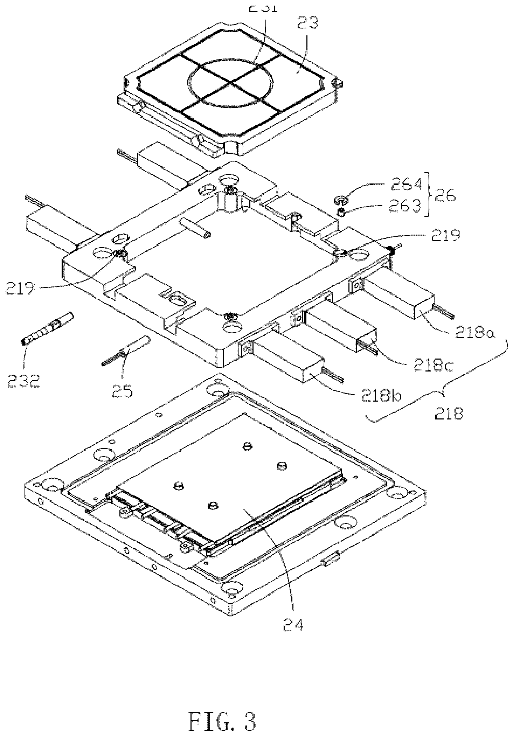

Referring to FIGS. 2 and 3, a fluid laying device 2 is shown according to a first embodiment of the present disclosure. The fluid laying device 2 of the embodiment can be used to lay fluid on the flow cell carrier 1a shown in FIG. 1a and the flow cell carrier 1b shown in FIG. 2. The fluid laying device 2 includes a manifold block 21, a support table 22, and a carrier mounting table 23. The carrier mounting table 23 is disposed at a center of the support table 22 for mounting the flow cell carrier. In the embodiment, the carrier mounting table 23 adsorbs the flow cell carrier onto the carrier mounting table 23 by vacuum adsorption. Outer surface of the base of the flow cell carrier functions as a mounting surface to be adsorbed on the carrier mounting table 23. A gas channel 231 is defined on the carrier mounting table 23, which is connected to an external vacuum source (not shown) through a vacuum adapter 232. In the embodiment, a temperature control module 24 is disposed under the carrier mounting table 23. The temperature control module 24 can be used when necessary to provide an appropriate temperature for fluid in the flow cell carrier mounted on the carrier mounting table 23. A temperature detector 25 is provided at a side of the temperature control module 24. The temperature detector 25 cooperates with the temperature control module 24 to facilitate a control device (not shown) to control the temperature of the fluid in the flow cell carrier.

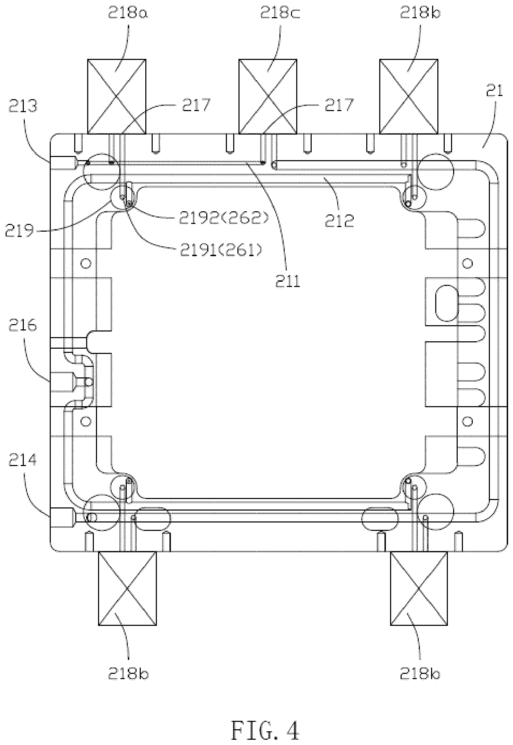

The manifold block 21 is disposed on the support table 22 and surrounds the carrier mounting table 23. In the embodiment, the manifold block 21 may be fixed to the support table 22 by mechanical fastening, such as by screw fastening or block clamping. Channels are defined in the manifold block 21. Referring to FIG. 4, the channels include a first channel 211 and a second channel 212. In addition, the manifold block 21 also defines a number of openings communicating with the interior channels. The openings include a fluid inlet 213, a first fluid outlet 214, a second fluid outlet 216, and a number of valve connection ports 217. The manifold block 21 is connected to a number of valve devices 218 through the valve connection ports 217. A control device can control the valve devices 218 to open or close a certain channel, thereby guiding the flow direction of the fluid. In the embodiment, the valve devices 218 include an inlet valve device 218a corresponding to the flow cell carrier inlet, an outlet valve device 218b corresponding to each outlet of the flow cell carrier, and a bypass valve device 218c between the inlet valve device 218a and one outlet valve device 218b. In the embodiment, the valve devices 218 may be two-way valves for example. The present disclosure does not limit the type of the valve device 218, and the valve device 218 may be other type of valve device, as long as the method is allowed to function.

Each outlet valve device 218b corresponds to a channel outlet of the flow cell carrier. The outlet valve devices 218b may correspond to the channel outlets of the flow cell carrier one-to-one. Taking the flow cell carrier 1a shown in FIG. 1a for fluid laying for example, there are three outlet valve devices 218b, which correspond to the three channel outlets 113a of the flow cell carrier 1. Taking the flow cell carrier 1a shown in FIG. 1b for fluid laying for example, there are three outlet valve devices 218b, which correspond to the three channel outlets 113b of the flow cell carrier 1b.

Carrier interfaces 219 are also provided on the manifold block 21. The carrier interfaces 219 correspond to the channel inlet and the channel outlets of the flow cell carrier one-to-one. Taking the flow cell carrier 1a shown in FIG. 1a for fluid laying for example, four carrier interfaces 219 are provided on the manifold block 21. One of the carrier interfaces 219 corresponds to the channel inlet 111a of the flow cell carrier 1a, and the other three carrier interfaces correspond to the channel outlets 113a of the flow cell carrier 1a one-to-one. Each carrier interface 219 defines a first hole 2191 and a second hole 2192. The first hole 2191 communicates with the inlet valve device 218a or with the outlet valve device 218b. The second hole 2192 communicates with the second channel 212. A sealing ring 26 is installed in each carrier interface 219. The sealing ring 26 defines a first hole 261 and a second hole 262. The first hole 261 communicates with the first hole 2191 of the carrier interface 219. The second hole 262 communicates with the second hole 2191 of the carrier interface 219. After the flow cell carrier 1 is placed on the mounting table 23, the openings of the flow cell carrier 1a are aligned with the sealing rings 26 one-to-one, and the first hole 261 of each sealing ring 26 communicates with the channel inlet 111a or with the channel outlet 113a of the flow cell carrier 1a. As such, the channel inlet 111a of the flow cell carrier 1a is connected to the first channel 211 through the first holes 2191 of the inlet carrier interface 219 and the inlet valve device 218A. Also, the channel outlet 113a of the flow cell carrier 1a is connected to the first channel 211 through the second hole 2192 of the carrier interface 219 and the outlet valve device 218b. Therefore, the fluid in the first channel 211 can enter the flow cell carrier 1a through the inlet valve device 218a, the first holes 2191 and 261, and the channel inlet 111a. Also, the fluid in the flow cell carrier 1a can enter the first channel 211 through the channel outlet, the first holes 261 and 2191, and the outlet valve device 218b. The second hole 262 of the sealing ring 26 communicates with the second channel 212 through the second hole 2192 of the carrier interface 219. In the embodiment, the sealing ring 26 includes a central portion 263 and a ring body 264 sleeved on the central portion 263. The first hole 261 is defined on the central portion 263. Edge of the ring body 264 abuts against a wall of the carrier interface 219 to seal the wall of the carrier interface 219. The ring body 264 has a notch, which is the second hole 262. The second hole 262 penetrates upper and lower sides of the ring body 264. The upper side of the ring body 264 faces the exterior of the carrier interface 219, and the lower side of the ring body 264 faces the interior of the carrier interface 219. The second hole 262 guides path of cleaning fluid and rectifies and limits the flow of the cleaning fluid. Thus, residue on the sealing ring 26 can be removed.

Through the channels defined in the manifold block 21, the inlet valve device 218a communicates with the first channel 211 and the first hole 261 of the corresponding sealing ring 26 installed at the inlet of the flow cell carrier. Through the channels defined in the manifold block 21, each outlet valve device 218b communicates with the first channel 211 and the first hole 261 of the corresponding sealing ring 26 installed at the outlet of the flow cell carrier. In the embodiment, the bypass valve device 218c is disposed on the first channel 211. Taking the bypass valve device 218c as a criterion, the first channel 211 includes a first section before the bypass valve device 218c and a second section after the bypass valve device 218c. The inlet valve device 218a is connected to the first section, and all the outlet valve devices 218b are connected to the second section. The bypass valve device 218c is connected to the two sections of the first channel 211 through the channels defined on the manifold block 21, thereby controlling the first channel 211 to be open or closed.

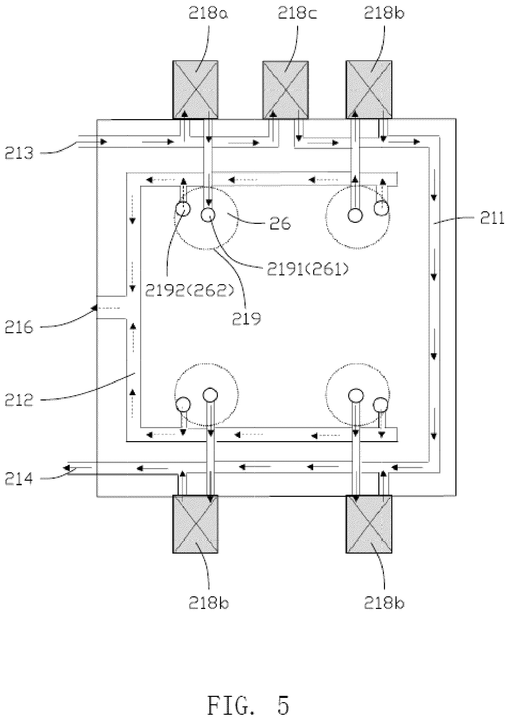

In the embodiment, one end of the first channel 211 communicates with the fluid inlet 213, the other end communicates with the first fluid outlet 214, and the center portion communicates with the bypass valve device 218c, thereby allowing the bypass valve device 218c to open or close the first channel 211. When the bypass valve device 218c is opened, the fluid flowing into the first channel 211 from the fluid inlet 213 can further flow out of the manifold block 21 through the first fluid outlet 214. In the embodiment, the fluid inlet 213 is a fluid inlet which can be used in common, through which reagents and the cleaning fluid to the manifold block 21 are supplied at different time periods. The first channel 211 is a common channel, through which the reagent and the cleaning fluid flow at different time periods. The second channel 212 is a channel for the cleaning fluid, which communicates with the second hole 2192 of the carrier interface 219 and the second fluid outlet 216. As shown in FIG. 5, the fluid entering the first channel 211 from the fluid inlet 213 can enter the flow cell carrier through the inlet valve device 218a, exit the outlet valve device 218b, and then flow through the first channel 211 and exit the fluid laying device 2 from the first fluid outlet 214. Thus, laying of fluid in the flow cell carrier is realized. In the present disclosure, a circuit for supplying fluid into and out of the flow cell carrier to achieve fluid laying in the flow cell carrier is called as a "fluid laying circuit". In the embodiment, the first channel 211 constitutes the fluid laying circuit. The fluid entering the first channel 211 from the fluid inlet 213 may pass through the bypass valve device 218c, then pass through the other section of the first channel 211, and finally be discharged out of the fluid laying device 2 from the first fluid outlet 214. Thus, excess fluid is discharged. The fluid entering the first channel 211 from the fluid inlet 213 may also enter the first hole 261 of the sealing ring 26 through the inlet valve device 218a, then enter the second channel 212 through the second hole 262, and be discharged out of the fluid laying device 2 from the second fluid outlet 216. Thus, some of channels and some the sealing rings 26 in the manifold block 21 are cleaned. The fluid entering the first channel 211 from the fluid inlet 213 may also enter the first hole 261 of the sealing ring 26 through any of the outlet valve devices 218b, then enter the second channel 212 through the second hole 262, and then be discharged out of the fluid laying device 2 from the second fluid outlet 216. In the present disclosure, a circuit for allowing fluid to flow through to clean relevant pipelines and components of the manifold block and/or the flow cell carrier is called as a "cleaning circuit". In the embodiment, the first channel 211 and the second channel 212 constitute the cleaning circuit. Flow direction of the fluids described above is achieved by controlling the inlet valve device 218a, the outlet valve devices 218b, and the bypass valve device 218c.

In actual use, the flow cell carrier is mounted on the mounting table 23. Each of the inlet and outlets of the flow cell carrier are pressed against the sealing ring 26 at a corresponding position. The first hole 261 of the sealing ring 26 is aligned with the inlet or outlet of the flow cell carrier. The fluid inlet 213 of the manifold block 21 is connected to an upstream pump (such as a syringe pump, not shown) through a valve device (not shown), and the second fluid outlet 216 is connected to a downstream pump (not shown) through another valve device (not shown). The upstream pump, the downstream pump, the inlet valve device 218a, the outlet valve devices 218b, and the bypass valve device are all connected to the control device, and are started or opened by the control device.

Taking the flow cell carrier 1a shown in FIG. 1a for fluid laying and cleaning for example, a fluid laying method and a cleaning method for subsequent cleaning of the fluid laying device 2 are as follows.

### Fluid laying process:

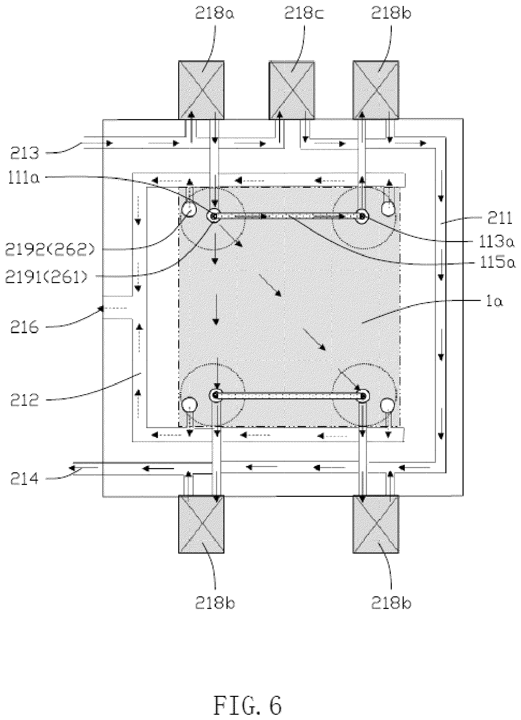

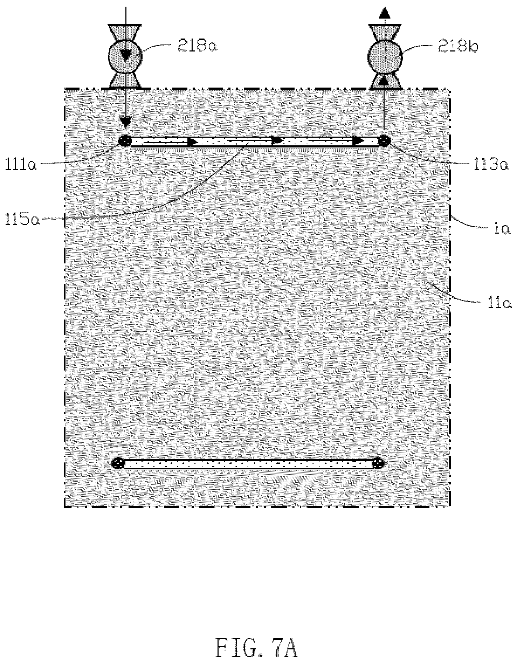

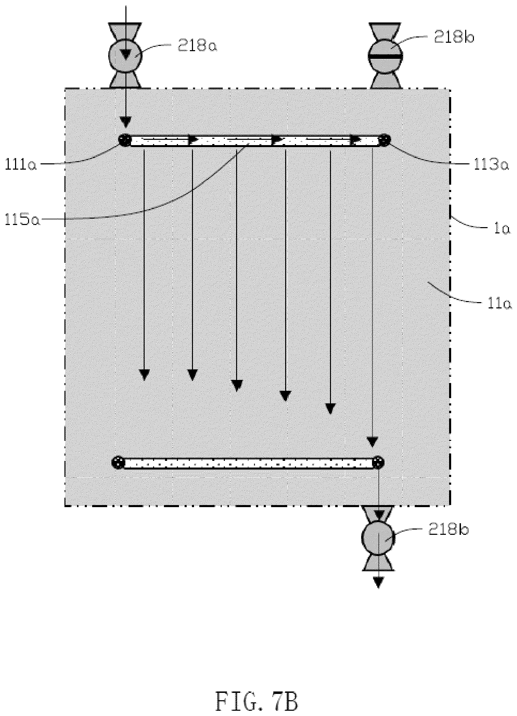

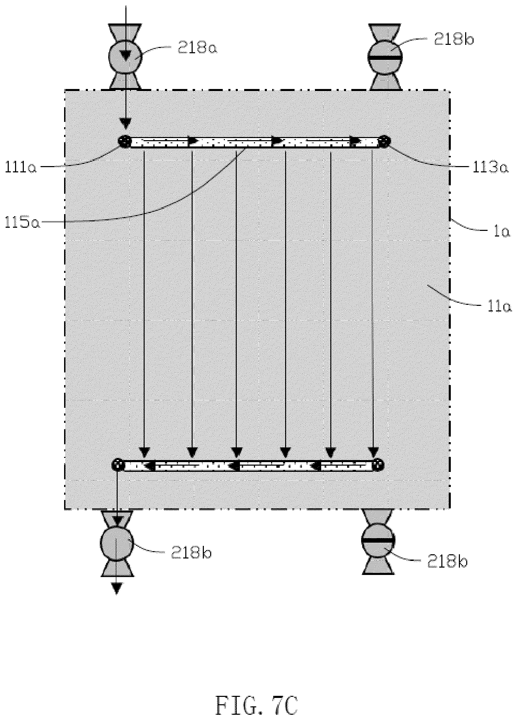

As shown in FIGS. 6 and 7A to 7C, after the flow cell carrier 1a is mounted on the mounting table 23, the inlet valve device 218a corresponds to the channel inlet 111a of the flow cell carrier 1a. The first outlet valve device 218b corresponds to the first channel outlet 113a, the second outlet valve device 218b corresponds to the second channel outlet 113a, and the third outlet valve device 218b corresponds to the third channel outlet 113a. During the fluid laying process, the upstream pump is first started, and the reagent functioning as the fluid is pushed to the fluid inlet 213 by the upstream pump. Next, the inlet valve device 218a and the bypass valve device 218c are controlled to open. The reagent flows through the first channel 211, so that the reagent fully infills between the fluid inlet 213 of the manifold block 21 and the channel inlet 111a of the flow cell carrier 1a. Then, the bypass valve device 218c is controlled to close and the first outlet valve device 218b is opened, and the upstream pump is controlled to continue pumping. Thus, the fluid enters the flow cell carrier 1a through the inlet valve device 218a and the channel inlet 111a, and further flows along the groove 115a toward the first channel outlet 113a. During such process, a portion of the fluid overflows from the groove 115a and forms a frontal blunt surface in the channel 11a. The first outlet valve device 218b is controlled to close and the second outlet valve device 218b is controlled to open, and the upstream pump is controlled to continue pumping. The fluid enters the channel 11a through the inlet valve device 218a and the channel inlet 111a, and further flows from the channel inlet 111a to the second channel outlet 113a. The second outlet valve device 218b is controlled to close and the third outlet valve device 218b is controlled to open, and the upstream pump is controlled to continue pumping. The fluid enters the channel 11a through the inlet valve device 218a and the channel inlet 111a, and flows from the channel inlet 111a to the third channel outlet 113a. In this way, laying of fluid uniformly is realized on the entire channel 11a of the flow cell carrier 1a. The residual fluid after the fluid laying process flows out from the first fluid outlet 214. In the above steps, for a fluid with low viscosity, the upstream pump can perform a single step of fluid pumping. For the fluid with higher viscosity, the upstream pump can perform fluid pumping in multiple steps.

### Cleaning process:

In order to avoid contamination during next laying of fluid, or influence of internal solutes on the components of the fluid laying device, the fluid residue at each channel/pipeline and the sealing ring 26 of manifold block 21 must be removed. One or more cleaning processes are carried out after the one or more fluid laying processes.

The flow cell carrier is removed before cleaning. In the cleaning process, the upstream pump is first started, and the cleaning fluid functioning as a fluid is pushed to the fluid inlet 213 by the upstream pump. Next, the bypass valve device 218c is controlled to open, and the cleaning fluid passes through the first channel 211 to the first fluid outlet 214, so that the downstream pipe between the first channel 211 and the manifold block 21 is filled with the cleaning fluid. The downstream pump is started to keep the cleaning circuit under a negative pressure, so that the cleaning fluid can be discharged. The third outlet valve device 218b is controlled to open, and the upstream pump is controlled to continue pumping fluid, so that the cleaning fluid is pushed out from the first hole 261 of the sealing ring 26 corresponding to the third outlet valve device 218b. The cleaning fluid drops into the second hole 262 of the sealing ring 26, and then passes through the second channel 212 and the second fluid outlet 216 and is collected by the downstream pump. The second outlet valve device 218b is controlled to open, and the upstream pump is controlled to continue pumping fluid, so that the cleaning fluid is pushed out from the first hole 261 of the sealing ring 26 corresponding to the second outlet valve device 218b, then dropping into the second hole 262 of the sealing ring 26, and then passing through the second channel 212 and the second fluid outlet 216 to be collected by the downstream pump. The first outlet valve device 218b is controlled to open, and the upstream pump is controlled to continue pumping fluid, so that the cleaning fluid is pushed out from the first hole 261 of the sealing ring 26 corresponding to the first outlet valve device 218b, drops into the second hole 262 of the sealing ring 26, and then passes through the second channel 212 and the second fluid outlet 216, being collected by the downstream pump. The inlet valve device 218a is controlled to open, and the upstream pump is controlled to continue pumping fluid, so that the cleaning fluid is pushed out from the first hole 261 of the sealing ring 26 of the corresponding inlet valve device 218A, falls into the second hole 262 of the sealing ring 26, and is collected by the downstream pump through the second channel 212 and the second fluid outlet 216. In the above cleaning method, the pressure source located upstream of the fluid laying device 2 (the upstream pump) drives the cleaning fluid with positive pressure to pass through and reach the designated pipeline and sealing ring. Then, a pressure source located downstream of the cleaning circuit (the downstream pump) collects and discharges the cleaning fluid. The cleaning method can also be repeated many times to achieve constant cleanliness.

Another fluid laying method of the present disclosure and another cleaning method for cleaning the fluid laying device 2 after the fluid laying process is described by taking the fluid laying and cleaning processes applied to the flow cell carrier 1b of FIG. 1b as an example.

### Fluid laying process:

After the flow cell carrier 1b is mounted on the mounting table 23, the inlet valve device 218a corresponds to the channel inlet 111b of the flow cell carrier 1b. The first outlet valve device 218b corresponds to the first channel outlet 113b, the second outlet valve device 218b corresponds to the second channel outlet 113b, and the third outlet valve device 218b corresponds to the third channel outlet 113b. During the fluid laying process, the upstream pump is first started, and the reagent as the fluid is pushed to the fluid inlet 213 by the upstream pump. Next, the bypass valve device 218c is controlled to open, and the reagent flows through the first channel, so that the reagent fully infills in between the fluid inlet 213 of the manifold block 21 and the channel inlet 111b of the flow cell carrier 1b. Then, the bypass valve device 218c is closed and the second outlet valve device 218b is controlled to open, and the upstream pump is controlled to continue pumping fluid. The reagent enters the flow cell carrier 1b through the inlet valve device 218a and the channel inlet 111b. The front edge of the reagent is fan-shaped. The reagent is finally guided to the second channel outlet 113b through the right angled L-shaped groove 115b. In this way, the laying of fluid is done uniformly on the entire channel 11b of the flow cell carrier 1b. The residual fluid after the fluid laying process flows out from the first fluid outlet 214 through the second outlet valve device 218b. In the above steps, for the fluid with a low viscosity, the upstream pump can perform a single step of fluid pumping. For the fluid with higher viscosity, the upstream pump can perform fluid pumping in multiple steps.

### Cleaning process:

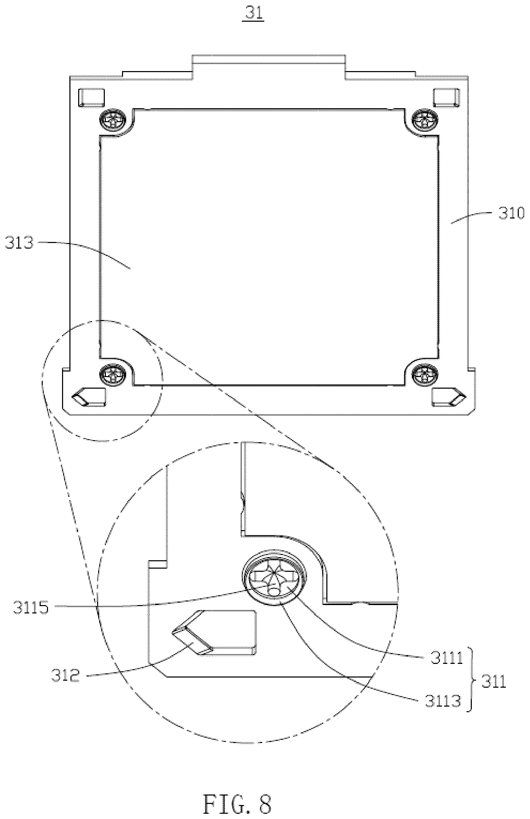

In the cleaning method currently described, an auxiliary cleaning tool is used to assist in the cleaning process. Referring to FIG. 8, a diagrammatic view of the auxiliary cleaning tool is shown according to a first embodiment of the present disclosure. In the embodiment, the end faces of the cleaning structures 311 of the auxiliary cleaning tool 31 press against the periphery of the inlet and outlet of the manifold block 21 for fluid to enter and exit the flow cell carrier.

The auxiliary cleaning tool 31 includes a body 310, the cleaning structures 311, positioning structures 312, and a mounting structure 313 disposed on the body 310. The mounting structure 313 is used to mount the body 310 to the fluid laying device 2. In the embodiment, the mounting structure 313 is a mounting surface corresponding to the carrier mounting table 23. In the embodiment where vacuum adsorption is used by the carrier mounting table 23 to fix objects, the mounting surface may be one suitable for adsorption. The positioning structures 312 are used for positioning purposes when a robot grips and installs the auxiliary cleaning tool 31 on the fluid laying device 2. In the embodiment, each positioning structure 312 has an indication for direction. The cleaning structures 311 correspond to the carrier interfaces 219 on the fluid laying device 2. In the embodiment, each cleaning structure 311 is generally cap-shaped, and includes a top portion 3111 and a flange portion 3113. The flange portion 3113 extends from the edge of the top portion 3111 toward the fluid laying device 2. The end face of the flange portion 3113 abuts against the periphery of the inlet and outlet of the manifold block 21 for fluid to enter and exit the flow cell carrier. In the embodiment, each cleaning structure 311 is fixed to the body 310 by adhesion or by mechanical clamping, and the body 310 defines a hole or a recess for receiving the cleaning structures 311. The entire cleaning structure 311 or only the flange portion 3113 is made of an elastic material or a deformable material, which may be rubber, silica gel, foam rubber, etc. A guiding structure 3115 is provided on the inner side of the top portion 3111 of each cleaning structure 311. The guiding structure 3114 is used to guide fluid from the middle area of the cleaning structure 311 to the edge area of the cleaning structure 311. When the auxiliary cleaning tool 31 is mounted to the fluid laying device 2 to assist the cleaning of the fluid laying device 2, the cleaning structures 311 correspondingly cover on the sealing rings 26 of the fluid laying device 2. The cleaning fluid from the first hole 261 of the sealing ring 26 is guided to the second hole 262 through the guiding structure 3115, enters the second channel 212, and then is collected and discharged by the downstream pump. In one embodiment, the guiding structure 3115 is a guiding groove. The guiding groove can be I-shaped, cross-shaped, or resembling the Union Jack flag.

The cleaning process using the auxiliary cleaning tool 31 to assist in cleaning is as follows.

First, the auxiliary cleaning tool 31 is mounted on the fluid laying device 2, causing the cleaning structures 311 of the auxiliary cleaning tool 31 to cover the sealing rings 26 of the fluid laying device 2 one-to-one. The upstream pump is started, and the cleaning fluid as the fluid is pushed to the fluid inlet 213 by the upstream pump. The bypass valve device 218c is controlled to open, and the cleaning fluid flows through the first channel 211 to the first fluid outlet 214, so that the downstream pipe between the first channel 211 and the manifold block 21 is filled with the cleaning fluid. The downstream pump is started to keep the cleaning circuit under a negative pressure, so that the cleaning fluid can be discharged. The third outlet valve device 218b is controlled to open, and the upstream pump is controlled to continue pumping fluid. Thus, the cleaning fluid is pushed out from the first hole 261 of the sealing ring 26 corresponding to the third outlet valve device 218b, is guided by the corresponding cleaning structure 311, flows into the second hole 262 of the sealing ring 26, and further passes through the second channel 212 and the second fluid outlet 216 to be collected by the downstream pump. The second outlet valve device 218b is controlled to open, and the upstream pump is controlled to continue pumping fluid. Thus, the cleaning fluid is pushed out from the first hole 261 of the sealing ring 26 corresponding to the second outlet valve device 218b, is guided by the corresponding cleaning structure 311 into the second hole 262 of the sealing ring 26, and further passes through the second channel 212 and the second fluid outlet 216 to be collected by the downstream pump. The first outlet valve device 218b is controlled to open, and the upstream pump is controlled to continue pumping fluid. Thus, the cleaning fluid is pushed out from the first hole 261 of the sealing ring 26 corresponding to the first outlet valve device 218b, is guided by the corresponding cleaning structure 311 into the second hole 262 of the sealing ring 26, and further passes through the second channel 212 and the second fluid outlet 216 to be collected by the downstream pump. The inlet valve device 218a is controlled to open, and the upstream pump is controlled to continue pumping fluid. Thus, the cleaning fluid is pushed out from the first hole 261 of the sealing ring 26 corresponding to the inlet valve device 218a, is guided by the corresponding cleaning structure 311 into the second hole 262 of the sealing ring 26, and further passes through the second channel 212 and the second fluid outlet 216 to be collected by the downstream pump.

A second embodiment of an auxiliary cleaning tool is also provided according to the present disclosure. The auxiliary cleaning tool of the second embodiment is generally similar to the auxiliary cleaning tool 31 of the first embodiment. The main difference is that after the auxiliary cleaning tool of the second embodiment is installed in the fluid laying device 2, gaps (not shown) exist between the cleaning structures of the auxiliary cleaning tool and the periphery of the inlet and outlet of the manifold block 21 for fluid to enter and exit the flow cell carrier. The gaps are used to balance the pipeline pressure between the cleaning circuit and the fluid circuit in the flow cell carrier. In the embodiment, each cleaning structure and the carrier interface 219 on the fluid laying device 2 may form a male-female connection structure. A one-way valve or a two-way solenoid valve (not shown) is provided downstream of the fluid laying circuit, that is, downstream of the first fluid outlet 214, which reduces or prevents the backflow of fluid in the downstream pipeline of the fluid laying device 2.

In the embodiment, the cleaning process assisted by the auxiliary cleaning tool is as follows.

First, the downstream pump of the cleaning circuit is started to keep the cleaning circuit in a negative pressure state, so that the cleaning fluid can be discharged. The auxiliary cleaning tool is installed on the fluid laying device 2, and the cleaning structures of the auxiliary cleaning tool correspondingly cover the sealing rings 26 of the fluid laying device 2 one-to-one. The upstream pump is started, and the cleaning fluid as the fluid is pushed to the fluid inlet 213 by the upstream pump. The bypass valve device 218c is controlled to open, and the one-way pump or two-way solenoid valve downstream of the fluid laying circuit is also controlled to open. The cleaning fluid flows through the first channel 211 and the first fluid outlet 214 toward the downstream pipe, so that the first channel and the downstream pipe of the manifold block 21 are filled with the cleaning fluid. The one-way valve or two-way solenoid valve downstream of the fluid laying circuit is controlled to close the downstream pipeline, thereby reducing or preventing the backflow of fluid. The third outlet valve device 218b is controlled to open, and the upstream pump is controlled to continue pumping fluid, so that the cleaning fluid is pushed out from the first hole 261 of the sealing ring 26 corresponding to the third outlet valve device 218b, is guided by the corresponding cleaning structure into the second hole 262 of the sealing ring 26, and further passes through the second channel 212 and the second fluid outlet 216 to be collected by the downstream pump. The second outlet valve device 218b is controlled to open, and the upstream pump is controlled to continue pumping fluid, so that the cleaning fluid is pushed out from the first hole 261 of the sealing ring 26 corresponding to the second outlet valve device 218b, is guided by the corresponding cleaning structure into the second hole 262 of the sealing ring 26, and further passes through the second channel 212 and the second fluid outlet 216 to be then collected by the downstream pump. The first outlet valve device 218b is controlled to open, and the upstream pump is controlled to continue pumping fluid, so that the cleaning fluid is pushed out from the first hole 261 of the sealing ring 26 corresponding to the first outlet valve device 218b, is guided by the corresponding cleaning structure 311 into the second hole 262 of the sealing ring 26, and further passes through the second channel 212 and the second fluid outlet 216 to be then collected by the downstream pump. The inlet valve device 218a is controlled to open, and the upstream pump is controlled to continue pumping fluid, so that the cleaning fluid is pushed out from the first hole 261 of the sealing ring 26 of the corresponding inlet valve device 218A, is guided by the corresponding cleaning structure into the second hole 262 of the sealing ring 26, and further passes through the second channel 212 and the second fluid outlet 216 to be then collected by the downstream pump.

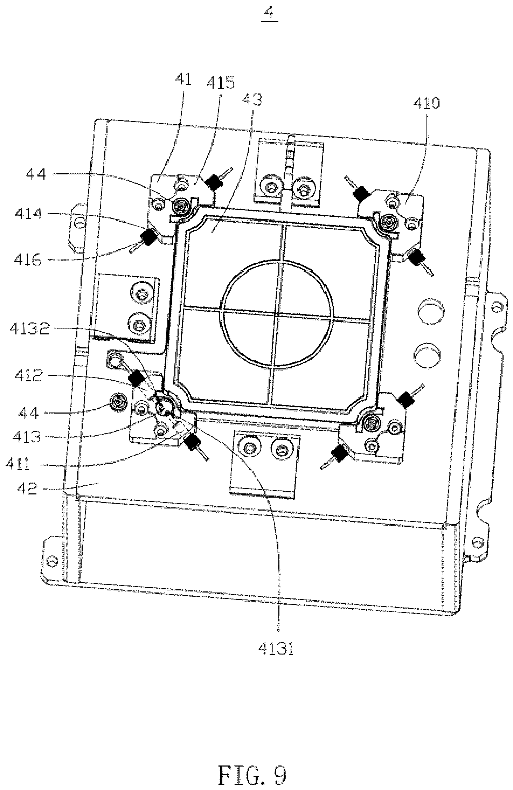

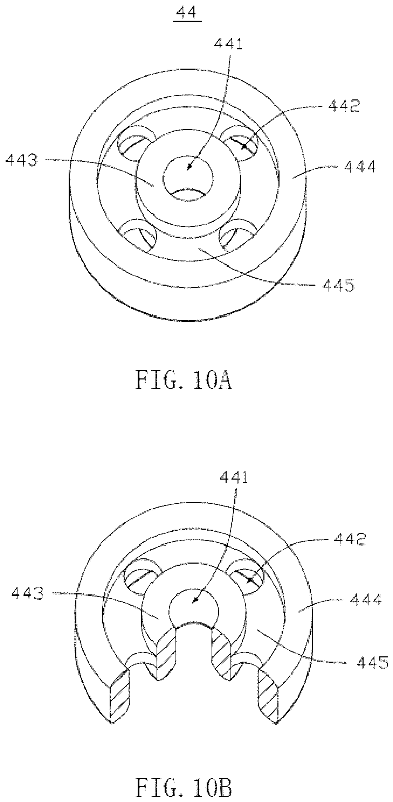

Referring to FIG. 9, a cleaning device for cleaning the channel inlet and channel outlet of a flow cell carrier is shown according to an embodiment of the present disclosure. The cleaning device 4 can be used together with the fluid laying device 2 to clean the areas around the channel inlet and the channel outlet of the flow cell carrier at a fixed frequency of cleaning, thereby maintaining the cleanliness of the mounting surface of the flow cell carrier. The cleaning device 4 includes a manifold block 41, a support table 42, and a carrier mounting table 43. The carrier mounting table 42 is arranged in the middle of the support table 41 for mounting the flow cell carrier. In the embodiment, the carrier mounting table 23 adsorbs the flow cell carrier on the carrier mounting table 43 by vacuum adsorption. Outer surface of the base of the flow cell carrier functions as a mounting surface to be adsorbed on the carrier mounting table 23. The manifold block 41 includes a plurality of sub-blocks 410 surrounding the carrier mounting table 23. Each sub-block 410 corresponds to the channel inlet or the channel outlet of the flow cell carrier. For example, the cleaning device 4 can be used to clean the flow cell carrier 1a shown in FIG. 1a. The manifold block 41 includes four sub-blocks 410 corresponding to the flow cell carrier 1a. One of the sub-blocks 410 corresponds to the channel inlet 111a of the flow cell carrier 1a. The other three sub-blocks 410 correspond to the flow outlets 113a of the flow cell carrier 1a. Each sub-block 410 defines a channel having two sections, that is, a first section 411 and a second section 412. Each sub-block 410 is further provided with a carrier interface 413 at a position facing the channel inlet 111a or the channel outlet 113a of the flow cell carrier 1a. The carrier interface 413 is used for mounting a sealing ring 44. Referring to FIG. 10, the sealing ring 44 defines a first hole 441 and a second hole 442. In the embodiment, the sealing ring 44 includes a central portion 443 as an inner ring and a ring body 444 as an outer ring. The outer side of the ring body 444 abuts against the wall of the carrier interface 413 to seal the wall of the carrier interface 413. The ring body 444 is spaced apart from the central portion 443 and connected to the central portion 443 by a connecting portion 445. The connecting portion 445 separates the space between the ring body 444 and the central portion 443 into upper and lower layers. The connecting portion 445 defines a hole, which allows communication of the upper and lower layers of the space with each other. The first hole 441 is defined in the central portion 443, and the hole of the connecting portion 443 is the second hole 442. The second hole 442 guides path of cleaning fluid and rectifies and limits the flow of the cleaning fluid. Thus, residue on the sealing ring 26 can be removed.

The bottom of the carrier interface 413 defines two holes, that is, a first hole 4131 and a second hole 4132. The first hole 4131 communicates with the first section 411 and the first hole 441 of the sealing ring 44. The second hole 4132 communicates with the second section 412 and the second hole of the sealing ring 44. The first section 411 communicates with the fluid inlet 414 defined on the manifold block 41, and the second section 412 communicates with the fluid outlet 415 defined on the sub-block 410. A joint 416 is installed on each of the fluid inlet 414 and the fluid outlet, and the upstream and downstream pipelines are connected to each other by the joint 416. Specifically, in the embodiment, the fluid inlet 414 of one of the sub-blocks 410 serves as the fluid inlet of the manifold block 41 as a whole, which is connected to an upstream cleaning fluid source through a pipeline. The fluid outlet 415 of another sub-block 410 serves as the fluid outlet of the manifold block 41 as a whole, this is connected to the downstream pump of the cleaning platform 4 through a pipeline. The sub-blocks 410 are connected in series with each other. The fluid outlet 415 of the previous sub-block 410 is connected to the fluid inlet 414 of the next sub-block 410. The fluid entering from the fluid inlet 414 of the manifold block 41 passes through the sub-blocks 410 in sequence, and flows out from the fluid outlet 415 of the manifold block 41. When the fluid passes through the carrier interface 413 of each sub-block 410, the fluid cleans the area around the inlet or outlet of the flow cell carrier.

The specific cleaning process is as follows:
After the fluid laying is completed or before the fluid laying is started, the flow cell carrier is transferred and installed to the cleaning device 4. Each of the channel inlet and channel outlets of the flow cell carrier is aligned with the first hole 441 of the sealing ring 44, and the sealing ring 44 seals the flow cell carrier. The downstream pump is started. After the cleaning fluid as the fluid enters from the cleaning fluid source to the fluid inlet 414 of the cleaning device 4, the cleaning fluid flows through the sub-blocks 410 in sequence and fills the cleaning circuit. Thus, the channel inlet and the channel outlets of the flow cell carrier are immersed and cleaned by the cleaning fluid. This cleaning process is applied for a period of time, for example, three to ten seconds, and then the downstream valve is closed to stop the cleaning process.

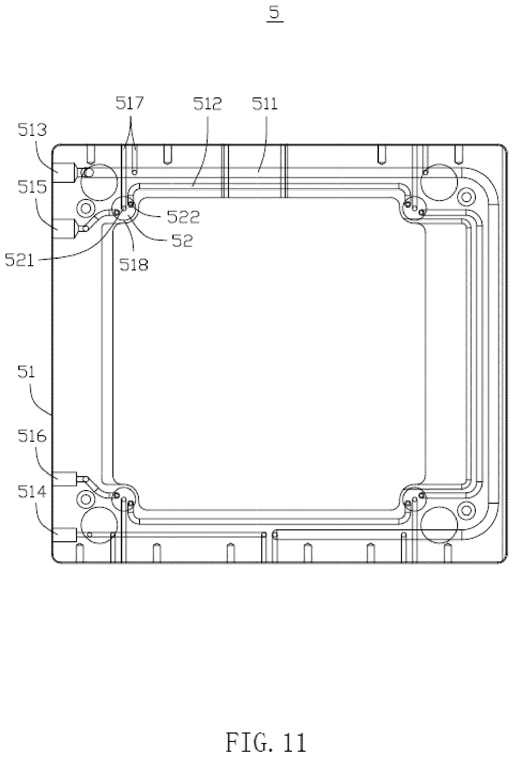

Referring to FIG. 11, a fluid laying device is shown according to a second embodiment of the present disclosure. The hardware structure of the fluid laying device 5 is basically the same as that of the fluid laying device 2. The difference is that in the fluid laying device 5, the cleaning circuit is used to clean around the channel inlet and the channel outlets of the flow cell carrier. The cleaning circuit and the fluid laying circuit for the reagent to enter and exit the flow cell carrier are independent of and do not communicate with each other.

The fluid laying device 5 includes a manifold block 51. The manifold block 51 defines a first channel 511 and a second channel 512. The first channel 511 allows a reagent to pass through for laying of fluid on the flow cell carrier, thereby forming a fluid laying circuit. The second channel 512 allows a cleaning fluid to pass through to clean the sealing ring 52 and the periphery of the channel inlet and the channel outlets of the flow cell carrier, thereby forming a cleaning circuit. The manifold block 51 defines a plurality of openings, including a first fluid inlet 513, a first fluid outlet 514, a second fluid inlet 515, a second fluid outlet 516, and a plurality of valve connection ports 517. The manner in which the valve connection ports are connected to a valve device (not shown) and the manner in which the fluid is controlled by the valve device to flow in the first channel 511 is the same as that of the fluid laying device 2 and will not be repeated. The first channel 511 communicates with the first fluid inlet 513 and the first fluid outlet 514, and further communicates with an upstream pump (not shown) through the first fluid inlet 513. The second channel 512 communicates with the second fluid inlet 515 and the second fluid outlet 516, and further communicates with a source of cleaning fluid (not shown) through the second fluid inlet 515 and a downstream pump (not shown) through the second fluid outlet 516. The manifold block 51 is provided with a carrier interface 518 corresponding to each of the channel inlet and the channel outlets of the flow cell carrier. Each carrier interface 518 is used to install a sealing ring 52. The structure of the sealing ring 52 is same as that of the sealing ring 44. In the embodiment, the second channel 512 is divided into a plurality of sections, and two adjacent sections are connected through the carrier interface 518. Specifically, a number of holes (not shown) are defined in the carrier interface 518. At least one of the holes communicates with the first hole 521 of the sealing ring 52, which is used to guide the fluid in the first channel 511 to enter the flow cell carrier through the first hole 521, or to guide the fluid in the flow cell carrier to enter the first channel 511 through the first hole 521. At least two of the holes are used to communicate with adjacent sections of the second channel 512 and also communicate with the second hole 522 of the sealing ring 52. Thus, the fluid in the second channel 512 passes through and cleans the sealing ring 52 and the periphery of the channel inlet and channel outlets of the flow cell carrier.

The fluid laying process on the flow cell carrier when using the fluid laying device 5 is as follows.

After the flow cell carrier is transferred and installed on the fluid laying device 5, each of the channel inlet and channel outlets of the flow cell carrier is aligned with the first hole 521 of the corresponding sealing ring 52. The sealing rings 52 seals the flow cell carrier. The fluid laying process is started, which can refer to the fluid laying process in other embodiments above and not repeated.

The cleaning process when using the fluid laying device 5 to clean the sealing ring 52 and the periphery of the channel inlet and the channel outlets of the flow cell carrier is as follows.

After the fluid laying is completed or before the next fluid laying is started, the downstream pump is started. After the cleaning fluid as the fluid from the cleaning fluid source enters the second fluid inlet 515 of the manifold block 51, the cleaning fluid flows through the sections of the second channel 512 in sequence and fills the cleaning circuit, so that the sealing ring 52 and the channel inlet and channel outlets of the flow cell carrier are immersed and cleaned by the cleaning fluid. The cleaning process is applied for a period of time, such as three to ten seconds, and then the downstream pump is closed to stop the cleaning process.

The fluid laying device 5 also includes a support table (not shown) and a carrier mounting table (not shown), the configurations and functions are those of the fluid laying device 2 and will not be repeated.

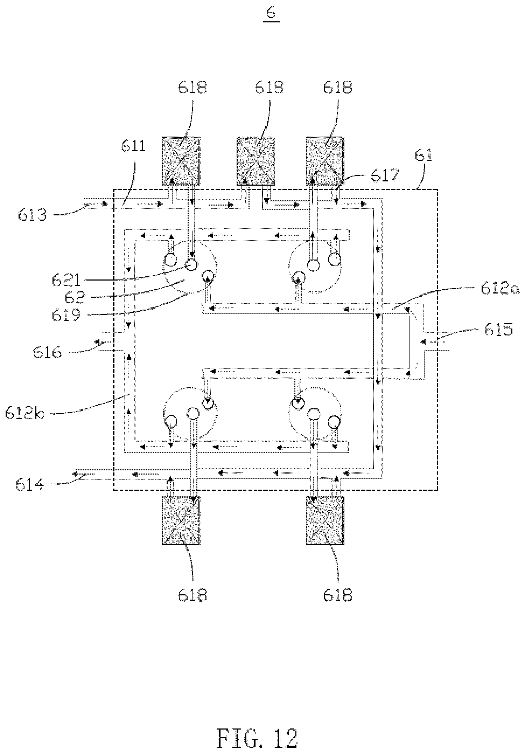

Referring to FIG. 12, a fluid laying device is shown according to a third embodiment of the present disclosure. The hardware structure of the fluid laying device 6 in the embodiment is basically the same as that of the fluid laying device 2, which also includes a support table, a carrier mounting table, and a manifold block. The configurations and functions of the support table and the carrier mounting table are the same as those of the fluid laying device 2 and will not be repeated. The difference between the fluid laying device 6 and the fluid laying device 2 is in the pipelines inside the manifold block. In the embodiment, the manifold block 61 of the fluid laying device 6 has three channels. The first channel 611 allows a reagent to pass through to realize fluid laying on the flow cell carrier, thereby forming a fluid laying circuit. The second channel 612a and the third channel 612b allow a cleaning fluid to pass through to clean the periphery of the channel inlet and the channel outlets of the flow cell carrier, thereby forming a cleaning circuit. The cleaning circuit and the fluid laying circuit are independent of and do not communicate with each other.

The manifold block 61 defines a number of openings, including a first fluid inlet 613, a first fluid outlet 614, a second fluid inlet 615, a second fluid outlet 616, and a number of valve connection ports 617. In the embodiment, the manner in which the valve connecting ports 617 is connected to the valve device 618 and the manner in which the fluid is controlled by the valve device to flow in the first channel 611 to achieve fluid laying are the same as those of the fluid laying device 2 and will not be repeated. Two ends of the first channel 611 communicate with the first fluid inlet 613 and the first fluid outlet 614, and further communicate with the upstream pump (not shown) and the reagent fluid source (not shown) through the first fluid inlet 613. The first channel 611 further communicates with the downstream waste fluid storage unit (not shown) through the first fluid outlet 614. The second channel 612a and the third channel 612b communicate with the second fluid inlet 615 and the second fluid outlet 616, respectively. The second fluid inlet 615 communicates with the upstream pump and the cleaning fluid source (not shown), and the second fluid outlet 616 communicates with the downstream pump (not shown) and the waste fluid storage unit (not shown). The manifold block 61 is provided with a carrier interface 619 corresponding to each of the channel inlet and the channel outlets of the flow cell carrier. The carrier interface 619 is used to install a sealing ring 62. The structure of the sealing ring 62 is the same as that of the sealing ring 44. The second channel 612a and the third channel 612b communicate with each other through the carrier interface 619. Specifically, a number of holes are defined in the carrier interface 619. At least one of the holes communicates with the first hole 621 of the sealing ring 62, and is used to guide the fluid in the first channel 611 to enter the flow cell carrier through the first hole 621, or to guide the fluid in the flow cell carrier to enter the first channel 611 through the first hole 621. At least two of the holes communicate with the second channel 612a and the third channel 612b. Thus, the cleaning fluid enters the carrier interface 619 from the second channel 612a, immerses and cleans the sealing ring 62 in the carrier interface 619 and the periphery of the channel inlet/outlet of the flow cell carrier, and then passes through the second channel 612b and exits the manifold block 61 through the second fluid outlet 616.

The fluid laying process on the flow cell carrier when using the fluid laying device 6 is as already described and will not be repeated.

The cleaning process of using the fluid laying device 6 to clean the sealing ring 62 and the periphery of the channel inlet and the channel outlet of the flow cell carrier is as follows:
After the fluid laying is completed or before the next fluid laying is started, the upstream pump and the downstream pump are started. After the cleaning fluid as the fluid from the cleaning fluid source enters the second fluid inlet 615 of the manifold block 61, the cleaning fluid flows through the second channel 612a, the carrier interface 619, and the third channel 612b in sequence, and fills the cleaning circuit, so that the sealing ring 52 and the periphery of the channel inlet and the channel outlets of the flow cell carrier are immersed and cleaned by the cleaning fluid. The cleaning process is applied for a period of time, three to ten seconds, and then the upstream pump and the downstream pump are turned off to stop the cleaning process. The flow cell carrier is removed. The downstream pump is started to discharge residual fluid at the sealing ring 62 through the third flow passage 612b and the second fluid outlet 616.

A fourth embodiment of a fluid laying device is also provided according to the present disclosure. In the fourth embodiment, the fluid laying device and the flow cell carrier are integrated as a whole, and the manifold block of the fluid laying device constitutes a cover of the flow cell carrier. Specifically, the fluid laying device includes a support table, a carrier mounting table, and a manifold block. The carrier mounting table can be a certain area on the support table. In the embodiment, the carrier mounting table is disposed in the central area of the support table, and a base of the flow cell carrier is disposed on such area. The manifold block is placed above the base, and a certain area of the manifold block faces and is spaced apart from the base. In the embodiment, the base and the manifold block are separated and hermetically sealed from each other. For example, a sealing fence is hermetically disposed between the base and the manifold block, so as to form a channel between the base and the manifold block. In the embodiment, the central area of the manifold block and the base constitute the flow cell carrier. The manifold block defines a channel inlet and a number of channel outlets. Grooves are defined between the channel inlet and one channel outlet and/or between two channel outlets, which are used to guide the fluid in the channel to realize fluid laying. The manifold block is also provided with a carrier interface at a position corresponding to each of the channel inlet and the channel outlets. The channel inlet and the channel outlets are disposed at the bottom of the corresponding carrier interfaces. A sealing ring is installed in the carrier interface. The sealing ring is the sealing ring 26 or can be sealing ring 44 in the above embodiments. The manifold block also defines a first channel and a second channel (and may further define a third channel). The first channel constitutes a fluid laying circuit, and the second channel (or the second channel and the third channel) constitutes a cleaning circuit. A number of valve devices are also provided outside the manifold block, to communicate with the first channel. The fluid laying in the flow cell carrier is controlled by controlling the valve devices to open or be closed. The setting of the channel in the manifold block, each opening on the manifold block, and the valve devices can refer to the above embodiments of the present disclosure. At least two holes are also provided in the carrier interface to connect to the first channel and the second channel. The configuration of the holes in the carrier interface can also refer to the above embodiments of the present disclosure. In the embodiment, the manifold block is fixed to the support table by mechanical fastening such as screw locking, clamp clamping, etc. When the manifold block is fixed to the support table, the manifold block also tightly fixes the base of the flow cell carrier.

The fluid laying method using the fluid laying device according to an embodiment can refer to the above descriptions and will not be repeated. The cleaning method of the pipelines and sealing rings in the fluid laying device can also refer to the above embodiments and will not be repeated.

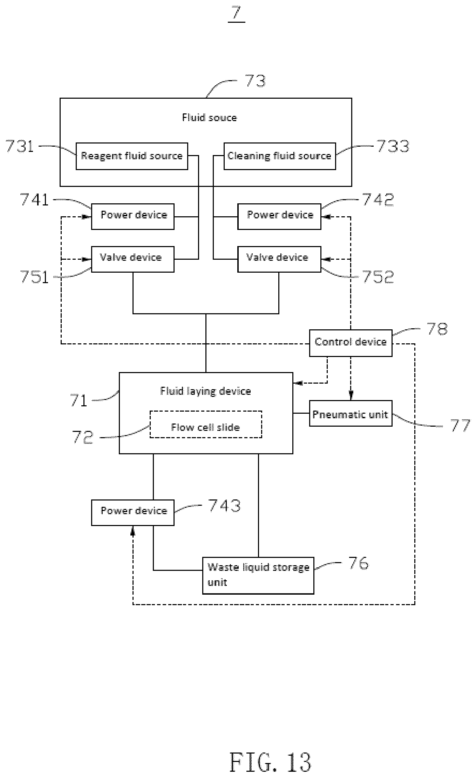

Referring to FIG. 13, a fluid laying system is shown according to a first embodiment of the present disclosure. The fluid laying system 7 in the embodiment includes a fluid laying device 71. The flow cell carrier 72 can be installed on or removed from the fluid laying device 71 as required. For example, during the fluid laying process, the flow cell carrier 72 needs to be installed on the fluid laying device 71. During the cleaning process, the flow cell carrier 72 can be kept on the fluid laying device 71 in a first case. At this time, the channel inlet and the channel outlet of the channel may also be cleaned together with the pipelines and the sealing rings of the fluid laying device 71. The flow cell carrier 72 may also be removed from the fluid laying device 71 in a second case, and at this time, only the pipes and the sealing rings in the fluid laying device 71 are cleaned. The fluid laying device 71 may be any of the fluid laying devices described in the above embodiments, such as the fluid laying device 2, 5, or 6.

A fluid source 73 is provided upstream of the fluid laying device 71. The fluid source 73 includes a reagent fluid source 731 and a cleaning fluid source 733. The reagent fluid source 731 is used to provide a reagent for the fluid laying device 71 to realize fluid laying on the flow cell carrier 72. The cleaning fluid source 733 is used to provide a cleaning fluid for cleaning the inner pipes of the fluid laying device 71, the sealing rings, and the periphery of the channel inlet and the channel outlets of the flow cell carrier 72. The reagent fluid source 731 communicates with a power device 741, which is used to provide power for the reagent to enter and flow through the fluid laying device 71 and the flow cell carrier 72. The power device 741 may be a pump. A valve device 751 is provided on the fluid path between the power device 741 and the fluid laying device 71. The valve device 751 is used to control the fluid path between the power device 741 and the fluid laying device 71 to open or be closed. The cleaning fluid source 733 communicates with a power device 742. The power device 742 is used to provide power for the cleaning fluid to enter the fluid laying device 71. The power device 741 may be a pump. A valve device 752 is provided between the power device 742 and the fluid laying device 71. The valve device 752 is used to control the power device 742 to connect to or disconnect from the fluid laying device 71.

A waste fluid storage unit 76 is provided downstream of the fluid laying device 71. The waste fluid storage unit 76 is used to store the waste fluid flowing out of the fluid laying device 71. A power device 743 is provided between the waste fluid storage unit 76 and the fluid laying device 71. The power device 743 may be a pump to provide power for the waste fluid in the fluid laying device 71 to flow into the waste fluid storage unit 76. In the embodiment, a pneumatic unit 77 is further provided in the fluid laying system 7, which is used to provide a low pressure (e.g., vacuum) to the carrier mounting table of the fluid laying device 71 to adsorb the flow cell carrier. The pneumatic unit 77 may be omitted in other embodiments in which the flow cell carrier is mounted to the fluid laying device 71 by mechanical fastening. A control device 78 is further provided in the fluid laying system 7, this may be a collection of hardware devices and software for controlling the power devices 741-743, the valve devices 751 and 752, the pneumatic unit 77, and the valve device of the fluid laying device 71. In one embodiment, the control device 78 may be a computer device including a processing unit, a storage unit, and computer programs. The computer programs are stored in the storage unit and executed by the processing unit, thereby allowing the control device 78 to control the power devices 741-743, the valve devices 751 and 752, the pneumatic unit 77, and the valve devices of the fluid laying device 71.

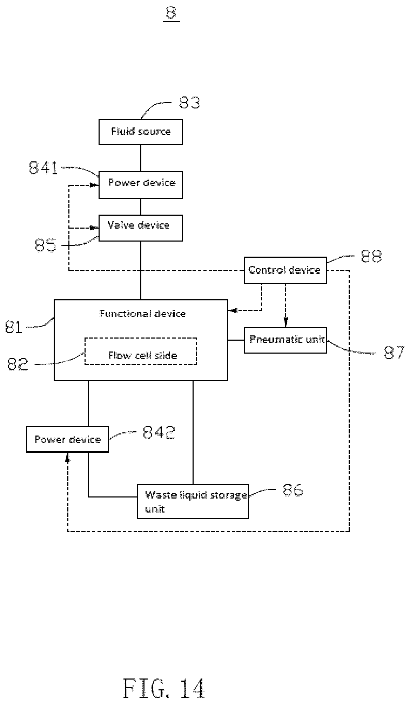

Referring to FIG. 14, a fluid laying system is shown according to a second embodiment of the present disclosure. The fluid laying system 8 in the embodiment can be used to perform the fluid laying process or the cleaning process. Therefore, compared with the fluid laying system 7 in the first embodiment, the fluid laying system 8 includes a functional device 81, which is a fluid laying device or a cleaning device. A flow cell carrier 82 is mounted on the functional device 81. A fluid source 83 is provided upstream of the functional device 81. The fluid source 83 can output a reagent or a cleaning fluid. A power device 84 and a valve device 85 are provided between the fluid source 83 and the functional device 81, in that order. The configurations downstream of the functional device 81 and other configurations are as for those of the fluid laying system 7 and will not be repeated.

The functional device 81 can be one of a fluid laying device and a cleaning device.

In practical use, the fluid laying device and method, the cleaning device and method, and fluid laying system provided in the present disclosure can be used in some links of molecular diagnosis or in vitro diagnosis requiring fluid laying and subsequent cleaning. For example, they can be applied to nucleic acid sequencing. Nucleic acid samples or reagents are evenly laid in the flow cell carrier. After the fluid laying process, pipes and sealing rings of the fluid laying device and/or the channel inlet and outlets of the flow cell carrier are cleaned.

The fluid laying device, fluid laying method, and fluid laying system provided in the present disclosure uses a positive pressure for laying of fluid to significantly improve the fluid laying speed and reduce the possibility of bubbles on the flow cell carrier. They can be applied to the fluid laying of flow cell carriers with large size and small aspect ratio, and can significantly improve the efficiency of replacement among reagents that enter the flow cell carriers in sequence and the uniformity of a fluid layer. The cleaning device and method provided increase the maintainability and reliability of the fluid laying device, significantly improve the service life of the device, and reduce the failure rate of the device.

It can be understood that some technical details or features of the above-mentioned embodiments of the fluid laying device and method, the cleaning device and method, and the fluid laying system of the present disclosure can be mutually referenced or replaced. For example, the sealing ring 26 of the fluid laying device 2 according to the first embodiment can be replaced by the sealing ring 44 of the cleaning device 4 from another embodiment.

It can be understood that although the above embodiments of the present disclosure describe the valve devices as being disposed on the outside of the manifold block, each valve device can also be disposed on the inside of the manifold block so long as the valve devices remain on the corresponding fluid path.

It can be understood that the above embodiments of the present disclosure describe the sealing ring as being installed in the carrier interface and the carrier interface and the sealing ring as independent components. In practice, the sealing ring can also be integrated with the corresponding carrier interface, or the sealing ring can form a part of the corresponding carrier interface.

It can be understood that the manifold block of the fluid laying device is a single unit when the fluid laying device is described in the above embodiment. In practice, the manifold block of the fluid laying device can also be composed of multiple sub-blocks. For example, the fluid laying device, when referring to the cleaning device, can have the manifold block composed of multiple sub-blocks, and the sub-blocks can be connected by pipes. The inlet valve devices and the outlet valve devices can be set on the corresponding sub-blocks. The bypass valve device can be set on a certain sub-block or on the pipeline connecting the sub-block.

It can be understood that the manifold block of the cleaning device is composed of sub-blocks in the descriptions of the cleaning device in the above embodiments. However, the manifold block of the cleaning device can also be a single unit. The cleaning circuit runs through the manifold block, and the fluid inlet and the fluid outlet are connected at both ends of the cleaning circuit.

It can be understood that the fluid laying device and the cleaning device described in the above embodiments can be described as fluid passage devices. Both of the fluid laying device and the cleaning device use a manifold block having a valve device and a carrier interface to control and guide the fluid. By providing a first hole only or a first hole and a second hole on the carrier interface, the fluid laying and the cleaning process can be realized. Specifically, the manifold block can be provided with a channel. The manifold block is provided with a fluid inlet, a fluid outlet, and a carrier interface communicating with the channel. The carrier interface is used to communicate with the channel in the flow cell carrier when the flow cell carrier is installed on the fluid passage device. The carrier interface has a first hole and a second hole. The second hole in the carrier interface is connected to the channel through a fluid path. The first hole in the carrier interface is connected to the channel through a fluid path, and is used to communicate with the channel in the flow cell carrier when the flow cell carrier is mounted on the fluid passage device. The fluid entering the channel from the fluid inlet enters and exits the flow cell carrier through the first hole of the carrier interface, and then flows out through the fluid outlet. Alternatively, the fluid entering the channel from the fluid inlet can enter the carrier interface through the first hole of the carrier interface, then enter the channel through the second hole of the carrier interface, and flow out from the fluid outlet. Alternatively, the fluid entering the channel from the fluid inlet further can enter and exit the carrier interface through the second hole, and then flow out through the fluid outlet.

It can be understood that the fluid laying device and the auxiliary cleaning tool described in the above embodiments can be described as a composite device. Before cleaning some components, the auxiliary cleaning tool is placed on the fluid laying device to assist in cleaning some components of the fluid laying device.

Finally, even though information and advantages of the present embodiments have been set forth in the foregoing description, together with details of the structures and functions of the present embodiments, the disclosure is illustrative only. Changes may be made in detail, especially in matters of shape, size, and arrangement of parts within the principles of the present exemplary embodiments, to the full extent indicated by the plain meaning of the terms in which the appended claims are expressed.

## Claims

1. A fluid laying device for laying fluid on a flow cell carrier, **characterized in that**, the fluid laying device comprises:
a manifold block, the manifold block defining a first channel, the manifold block comprising a first fluid inlet communicating with the first channel, a first fluid outlet, and a plurality of carrier interfaces;
wherein each of the plurality of carrier interfaces defines a first hole, the first hole of each of the plurality of carrier interfaces is configured to connect one of a channel inlet and a channel outlet of the flow cell carrier;
wherein an inlet valve device, a bypass valve device, and a plurality of outlet valve devices are provided on the manifold block; the inlet valve device and the plurality of outlet valve devices correspond to the plurality of carrier interfaces one-to-one, and are disposed on fluid paths from the corresponding carrier interfaces to the first channel for opening or closing the fluid paths;
wherein the first channel comprises a first section and a second section, the bypass valve device is between the first section and the second section to control connection or disconnection between the first section and the second section, the inlet valve device is connected to the first section, and the plurality of outlet valve devices is connected to the second section.

2. The fluid laying device according to claim 1, **characterized in that**, the fluid laying device further comprises a support table and a carrier mounting table, the carrier mounting table and the manifold block are disposed on the support table, and the carrier mounting table is configured to mount the flow cell carrier.

3. The fluid laying device according to claim 2, **characterized in that**, the carrier mounting table is configured to adsorb the flow cell carrier by vacuum adsorption or low-pressure adsorption, and/or, the manifold block surrounds the carrier mounting table.

4. The fluid laying device according to claim 1, **characterized in that**, the manifold block further defines a second channel, the manifold block further comprises a second fluid outlet communicating with the second channel, each of the plurality of carrier interfaces defines a second hole, and the second hole of each of the plurality of carrier interfaces is configured to communicate with the second channel.

5. The fluid laying device according to claim 4, **characterized in that**, the plurality of carrier interfaces disconnecting from the channel inlet and the channel outlet of the flow cell carrier allows a fluid in the first channel to pass through the first hole of the corresponding carrier interface and enter the second hole of the corresponding carrier interface, and to finally flow out from the second fluid outlet through the second channel.

6. The fluid laying device according to claim 4, **characterized in that**, the manifold block further defines a second fluid inlet communicating with the second channel, each of the plurality of carrier interfaces comprises at least two second holes, the second channel is divided into a plurality of sections by the plurality of carrier interfaces, adjacent two of the plurality of sections communicate with each other through the second holes of the corresponding carrier interface, thereby allowing the fluid from the second fluid inlet to flow out from the second fluid outlet after passing through the plurality of carrier interfaces in sequence.

7. The fluid laying device according to claim 1, **characterized in that**, the manifold block further defines a second channel and a third channel, the manifold block further comprises a second fluid inlet communicating with the second channel and a second fluid outlet communicating with the third channel, each of the plurality of carrier interfaces has at least two second holes, two of the at least two second holes communicate with the second channel and the third channel, thereby allowing a fluid from the second fluid inlet to flow out from the second fluid outlet after passing through the plurality of carrier interfaces in sequence.

8. The fluid laying device according to any one of claims 4 to 7, **characterized in that**, a sealing ring is arranged in each of the plurality of carrier interfaces, and each sealing ring defines a first hole communicating the first hole of the corresponding carrier interface.

9. The fluid laying device according to claim 8, **characterized in that**, each sealing ring further defines a second hole communicating with the second hole of the corresponding carrier interface, the second hole of the sealing ring allows the fluid from the first hole of the sealing ring to enter the second hole of the corresponding carrier interface.

10. The fluid laying device according to claim 9, **characterized in that**, each sealing ring comprises a central portion and a ring body sleeved on the central portion, the ring body abuts against a wall of the corresponding carrier interface, the first hole of the sealing ring is defined on the central portion, and the second hole is defined on the ring body and communicates an upper side and a lower side of the ring body with each other.

11. The fluid laying device according to claim 9, **characterized in that**, each sealing ring comprises a central portion, a ring body, and a connecting portion connecting the ring body and the central portion, the ring body abuts against a wall of the corresponding carrier interface, the first hole of the sealing ring is defined on the central portion, and the second hole is defined on the connecting portion and communicates an upper side and a lower side of the connecting portion with each other.

12. The fluid laying device according to any one of claims 1 to 11, **characterized in that**, the fluid laying device further comprises the flow cell carrier, the flow cell carrier comprises a base and a cover, a channel is formed between the base and the cover, and a portion of the manifold block constitutes the cover.

13. The fluid laying device according to claim 12, **characterized in that**, the portion of the manifold block constituting the cover defines the channel inlet and the channel outlet, and the channel inlet and the channel outlet communicate with the channel.

14. The fluid laying device according to claim 13, **characterized in that**, at least one groove for rectification of fluid is defined on a side of the manifold block facing the base, the at least one groove communicates with the channel inlet and at least one of the channel outlet, and/or the groove communicates with a portion or all of the channel outlet.

15. The fluid laying device according to claim 14, **characterized in that**, the portion of the manifold block constituting the cover defines one channel inlet and three channel outlets, any three of the fluid inlet and the fluid outlets are not on a same straight line.

16. The fluid laying device according to claim 15, **characterized in that**, the flow cell carrier is square, and the channel inlet and the channel outlets are distributed at four corners of the flow cell carrier.

17. The fluid laying device according to claim 16, **characterized in that**, the at least one groove for rectification of fluid comprises two grooves, one of the two grooves communicates with the channel inlet and one of the channel outlets adjacent to the channel inlet, and the other of the two grooves communicates with another of the channel outlets adjacent to the channel inlet and a remaining channel outlet disposed diagonally opposite the channel inlet; or, the at least one groove for rectification of fluid is L-shaped and communicates with the three channel outlets.

18. The fluid laying device according to any one of claims 1 to 11, **characterized in that**, the manifold block comprises a plurality of sub-blocks, and each of the plurality of sub-block communicates with the corresponding channel through a pipeline.

19. The fluid laying device according to any one of claims 1 to 18, **characterized in that**, the fluid laying device is configured to lay a nucleic acid sample or a reagent in the flow cell carrier.

20. A fluid laying method configured to control a fluid laying device according to claim 1 to introduce a fluid into a channel in a flow cell carrier, **characterized in that**, the method comprises:
controlling a power device to start, thereby allowing the power device to push the fluid into the fluid inlet of the fluid laying device;
controlling the bypass valve device to open, thereby allowing the fluid to flow from the fluid inlet to the fluid outlet;
controlling the bypass valve device to close; and
controlling at least one of the inlet valve device and the plurality of outlet valve devices to open, thereby allowing the fluid to enter and flow through the channel in the flow cell carrier.

21. The fluid laying method according to claim 20, **characterized in that**, a different one of the plurality of outlet valve devices is controlled to open or the plurality of outlet valve devices is controlled to open in sequence, thereby controlling a direction of the fluid in the flow cell carrier, so that the fluid lays at all positions of the channel in sequence.

22. The fluid laying method according to claim 21, **characterized in that**, a corresponding one of the plurality of outlet valve devices is controlled to open or the plurality of outlet valve devices is controlled to open in a specific order, according to distribution and extension direction of grooves for rectification of fluid in the flow cell carrier.

23. The fluid laying method according to claim 22, **characterized in that**, in two grooves for rectification of fluid in the flow cell carrier, a first groove communicates with the channel inlet and one of the channel outlets adjacent to the channel inlet, a second groove communicates with another of the channel outlets adjacent to the channel inlet and a remaining channel disposed diagonally opposite to the channel inlet, the specific order of controlling the plurality of outlet valve devices to open comprises: first controlling the outlet valve device corresponding to the channel outlet communicating with the first groove to open; then controlling the outlet valve device corresponding to the channel outlet disposed diagonally opposite to the channel inlet to open; finally controlling the outlet valve device corresponding to the channel outlet communicating with the second groove to open.

24. The fluid laying method according to claim 22, **characterized in that**, when the L-shaped groove in the flow cell carrier communicates with the three channel outlets, one of the plurality of outlet valve devices controlled to be open is the outlet valve device corresponding to the channel outlet at an apex position of the L-shaped groove.

25. The fluid laying method according to any one of claims 20 to 24, **characterized in that**, the fluid laying method is configured to spread a nucleic acid sample or a reagent in the flow cell carrier.

26. The fluid laying method according to any one of claims 20 to 25, **characterized in that**, the fluid laying method uses a positive pressure to push the fluid to realize fluid laying, and uses a negative pressure to collect a waste fluid.

27. A fluid laying system, comprising:
a fluid laying device according to any one of claims 1 to 19;
a first power device configured to drive a fluid from a fluid source to enter and flow in the fluid laying device;
a valve device disposed between the power device and the fluid laying device, and configured to open or close a fluid path between the power device and the fluid laying device; and
a control device configured to control the power device, the valve device, and the inlet valve device and the plurality of outlet valve devices in the fluid laying device.

28. The fluid laying system according to claim 27, **characterized in that**, further comprises a second power device, the second power device is arranged downstream of the fluid laying device, and is configured to provide power for outflow of the waste fluid in the fluid laying device.

29. A composite device, **characterized in that**, comprises:
a fluid laying device according to any one of claims 3 to 11; and
an auxiliary cleaning tool, the auxiliary cleaning tool comprising a plurality of cleaning structures, each of the plurality of cleaning structures configured to align with and install on one carrier interface of the fluid laying device, and guide the fluid in the first hole of the carrier interface into the second hole of the carrier interface.

30. The composite device according to claim 29, **characterized in that**, the auxiliary cleaning tool further comprises a body, the plurality of cleaning structures is provided on the body, when the body is installed on the fluid laying device, the plurality of cleaning structures corresponds to the plurality of carrier interfaces.

31. The composite device according to claim 30, **characterized in that**, the cleaning structure is cap-shaped, and comprises a top portion and a flange portion extending from an edge of the top portion toward the fluid laying device, a guiding structure is provided on an inner side of the top portion for guiding the fluid flowing out of the first hole of the carrier interface into the second hole of the carrier interface; or, a guiding groove is defined on an inner side of the top portion for guiding the fluid flowing out of the first hole of the carrier interface into the second hole of the carrier interface; or, a guiding groove being I-shaped, cross-shaped, or resembling the Union Jack flag is defined on an inner side of the top portion for guiding the fluid flowing out of the first hole of the carrier interface into the second hole of the carrier interface.

32. The composite device according to claim 31, **characterized in that**, the cleaning device is fixed to the body by adhesion or engagement.

33. A fluid passage device, **characterized in that**, comprises:
a manifold block, the manifold block defining a channel, the manifold block comprising a fluid inlet communicating with the channel, a fluid outlet, and a plurality of carrier interfaces; the plurality of carrier interfaces configured to communicate a channel in the flow cell carrier when the flow cell carrier is installed on the fluid passage device;
wherein each of the plurality of carrier interfaces has a first hole and a second hole, the second hole in each of the plurality of carrier interfaces is connected to the channel through a fluid path, the first hole in each of the plurality of carrier interfaces is connected to the channel through another fluid path, the first hole is configured to communicate with the channel in the flow cell carrier when the flow cell carrier is mounted on the fluid passage device;
wherein the fluid entering the channel from the fluid inlet enters and exits the flow cell carrier through the first hole of each of the plurality of carrier interfaces, and then flows out through the fluid outlet; or, the fluid entering the channel from the fluid inlet enters the plurality of carrier interfaces through the first holes of the plurality of carrier interfaces, then enters the channel through the second holes of the plurality of carrier interfaces, and flows out from the fluid outlet; or, the fluid entering the channel from the fluid inlet enters and exits the plurality of carrier interfaces through the second holes, and then flows out through the fluid outlet.

34. The fluid passage device according to claim 23, **characterized in that**, a sealing ring is provided in each of the plurality of carrier interfaces, the sealing ring defines a first hole and a second hole, the first hole of the sealing ring communicates with the first hole of the corresponding carrier interface, and is used to communicate with the channel in the flow cell carrier, the second hole of the sealing ring allows the fluid from the first hole of the sealing ring to enter the second hole of the corresponding carrier interface.

35. The fluid passage device according to claim 34, **characterized in that**, the sealing ring comprises a central portion and a ring body sleeved on the central portion, the ring body abuts against a wall of the corresponding carrier interface, the first hole of the sealing ring is defined on the central portion, the second hole is defined on the ring body and communicates an upper side and a lower side of the ring body with each other.

36. The fluid passage device according to claim 34, **characterized in that**, the sealing ring comprises a central portion, a ring body, and a connecting portion connecting the ring body and the central portion, the ring body abuts a wall of the corresponding carrier interface, the first hole of the sealing ring is defined on the central portion, the second hole is defined on the connecting portion and communicates an upper side and a lower side of the connecting portion with each other.

37. The fluid passage device according to claim 33, **characterized in that**, the channel comprises a first channel communicating with the fluid inlet and a second channel communicating with the fluid outlet, a first hole of the carrier interface communicates with the first channel, and a second hole of the carrier interface communicates with the second channel.

38. The fluid passage device according to claim 33, **characterized in that**, the fluid outlet comprises a first fluid outlet and a second fluid outlet, the channel comprises a first channel communicating with the fluid inlet and the first fluid outlet and a second channel communicating with the second fluid outlet, the first hole of the carrier interface communicates with the first channel, the second hole of the carrier interface communicates with the second channel.

39. The fluid passage device according to claim 33, **characterized in that**, the fluid inlet comprises a first fluid inlet and a second fluid inlet, the fluid outlet comprises a first fluid outlet and a second fluid outlet, the channel comprises a first channel communicating the first fluid inlet and the first fluid outlet, and a second channel communicating the second fluid inlet and the second fluid outlet, the first hole of the carrier interface communicates with the first channel, the second hole of the carrier interface communicates with the second channel.

40. The fluid passage device according to claim 33, **characterized in that**, the fluid inlet comprises a first fluid inlet and a second fluid inlet, the fluid outlet comprises a first fluid outlet and a second fluid outlet, the channel comprises a first channel communicating the first fluid inlet and the first fluid outlet, a second channel communicating the second fluid inlet, and a third channel communicating the second fluid outlet, the first hole of the carrier interface communicates with the first channel, each of the plurality of carrier interface comprises a plurality of second holes, at least one of the plurality of second holes of the carrier interface communicates with the second channel, at least another of the plurality of second holes communicates with the third channel.

41. The fluid passage device according to claim 33, **characterized in that**, different sections of at least one of the channel communicate with each other via the second hole of the carrier interface.

42. The fluid passage device according to claim 33, **characterized in that**, one of the channel comprises at least two sections, each of the plurality of carrier interface comprises a plurality of second holes, at least one of the plurality of second holes of the carrier interface communicates with one of the sections, and at least another of the plurality of second holes of the carrier interface communicates with another of the sections.

43. The fluid passage device according to claim 33, **characterized in that**, the manifold block comprises a plurality of sub-blocks, and each of the plurality of sub-blocks communicates with the corresponding channel through a pipeline.
